# EUROPEAN PATENT APPLICATION

(11) **EP 3 973 950 A1**
(43) Date of publication of application: **30.03.2022**
(21) Application number: 20198426.7
(22) Date of filing: 25.09.2020
(51) Int. Cl.: A61K 9/00, A61K 31/198, A61K 31/4415, A61K 31/455, A61K 31/525, A61K 31/714, A61P 43/00, A61K 9/08, A61K 9/10, A61K 47/44

(54) **PARENTERAL NUTRITION FORMULATION COMPRISING VITAMIN B12**

(71) Applicant: Baxter International Inc, Deerfield, IL 60015 (US); BAXTER HEALTHCARE SA, 8152 Glattpark (Opfikon) (CH)
(72) Inventor: DUPONT, Caroline, 1050 Ixelles (BE); BOUREZG, Zouaoui, 1420 Braine l Alleud (BE); COUTHOUIS, Aurélie, B-1480 Tubize (BE)
(74) Representative: Hertin und Partner Rechts- und Patentanwälte PartG mbB

(57) **Abstract**

The invention relates to a terminally heat-sterilized multi-chamber container for parenteral nutrition, comprising a first chamber containing a macronutrient formulation and a second chamber comprising a vitamin B12 formulation, wherein the vitamin B12 formulation (a) is essentially free from glucose, amino acids, vitamin B1, ascorbyl palmitate and/or vitamin C; and (b) stably comprises vitamin B12 for at least 6 months at a temperature of from 1°C to 40°C and/or for at least 24 months at a temperature of 25°C.

## Description

The invention relates to the field of clinical nutrition, corresponding medical products and nutritional solutions.

The invention relates to a medical product for preventing or correcting vitamin B12 deficiency in a patient. The invention therefore relates to a terminally heat sterilized multi-chamber container for parenteral nutrition, comprising a first chamber containing a macronutrient formulation and a second chamber comprising a vitamin B12 formulation, wherein the vitamin B12 formulation (a) is essentially free from glucose, amino acids, vitamin B1 and/or vitamin C, and (b) stably comprises vitamin B12 for at least 6 months at a temperature of from 1°C to 40°C and/or for at least 24 months at a temperature of 25°C.

In embodiments of the invention, the terminal heat sterilization process has a Co value of no more than 130 minutes, preferably no more than 120 minutes, preferably no more than 100 minutes. Furthermore, in embodiments the formulation does not comprise trace elements, cysteine and/or amino acids.

### BACKGROUND OF THE INVENTION

Parenteral Nutrition aims to supply nutrients to patients by venous access. Nutrients are composed of macronutrients (lipids, amino acid or protein and dextrose or carbohydrates), micronutrients (vitamins and trace elements) and electrolytes.

Parenteral nutrition, such as in the form of one or more solutions, can be provided in the form of flexible bags, either in the form of single flexible bags containing glucose, amino acids or lipids with or without electrolytes, which can be mixed together prior administration, or multi chamber flexible bags providing separated macronutrients and electrolytes, in a ready to use format. The bags are typically made of a synthetic or plastic material, for example materials such as polypropylene (PP), polyethylene (PE), ethylene vinyl alcohol (EVOH), ethylene-vinyl acetate (EVA) and all possible copolymers, essentially any synthetic material suitable for containing the components to be administered.

In the state of the art, micronutrients are typically added to nutrition bags directly before administration. For this purpose, vitamins can be provided in glass vials in the form of lyophilizates or solutions to be reconstituted and/or mixed into the nutrition/infusion bags. Trace elements are also provided in glass vials or polypropylene ampules meant to be mixed into infusion bags prior to administration.

Prior to usage, referring to the start administering the formulation to the patient, the micronutrients are sometimes added to the mixture or macronutrients via an injection port of the container or bag (septum) or are added via a Y-connector to the infusion line. This process takes time and several handling steps increasing the risk of errors or contamination.

The major reason for adding reconstituted micronutrients and in particular vitamins that have been reconstituted from lyophilized formulations to macronutrients or the mixture directly before administration is that some vitamins have significant stability issues when provided in liquid ready-to-use products. Vitamin A and vitamin C are two major examples (Ferguson et al. A Review of Stability Issues Associated with Vitamins in Parenteral Nutrition; Clinical Nutrition ESPEN 2014, vol.9 issue 2). However, as vitamin A is regarded as an essential vitamin required in total PN (TPN), it must be a component of an "all-in-one" ready-to-use formulation.

Vitamin B12, also known as cobalamin, is a water-soluble vitamin involved in the metabolism of every cell of the human body. It is a cofactor in DNA synthesis, and in both fatty acid and amino acid metabolism. It is particularly important in the normal functioning of the nervous system via its role in the synthesis of myelin, and in the maturation of developing red blood cells in the bone marrow. Vitamin B12 exists in four near-identical chemical forms (vitamers): cyanocobalamin, hydroxocobalamin, adenosylcobalamin and methylcobalamin. Cyanocobalamin and hydroxocobalamin are used to prevent or treat vitamin deficiency; once absorbed they are converted into adenosylcobalamin and methylcobalamin, which are the forms which have physiological activity. Therefore, as used herein in the context of the medical product of the invention for preventing or correcting vitamin B12 deficiency in a patient, the term vitamin B12 relates to the forms cyanocobalmin and hydroxocobalmin.

Vitamin B12 is generally considered to be stable under most food processing operations, but like all water-soluble vitamins, it is subject to large losses upon heating of up to 100° C (Leskova et al. Journal of Food Composition and Analysis; 19 (2006), 252-276). The most important associated compound with vitamin B12 activity is cyanocobalamin, which is decomposed by both oxidizing and reducing agents. In neutral and weak acid solutions, it is relatively stable to both atmospheric oxygen and heat. It is only slightly stable in alkaline solutions and strong acids. The stability of vitamin B12 is significantly influenced by the presence of other vitamins. Vitamin B12 is normally stable during pasteurization, but is instable during sterilization, which is usually associated with strong losses of Vitamin B12. Accordingly, it is difficult to stabilize vitamin B12 in sterilized nutritional products for parenteral nutrition.

There is no terminally heat-sterilized medical product available which stably comprises vitamin B12 for the treatment or prevention of vitamin B12 deficiency in a patient. In particular, there is no terminally heat-sterilized TPN product available at the moment that provides pre-packaged, prestored vitamin B12 in a ready to use liquid formulation for injection that is sufficiently stable for prolonged storage, such as 6, 12 or even 24 months. There is especially no terminally heat-sterilized all-in-one (AIO) or multi-chamber bag (MCB) product for parenteral nutrition containing all the required nutritional components, such as macronutrients (carbohydrates, fats, amino acids) and micronutrients (vitamins, trace elements). So far, it has not been possible to stabilize vitamin B12 in such standard parenteral nutrition products, which contain a variety of different compounds in flexible bags, and which underwent terminal heat sterilization. Known problems with vitamin B12 in such products encompass the heat-sensitivity of vitamin B12, which makes it difficult to make it a component of said AIO or MCB product which generally have a relatively high volume and therefore require a higher exposure to be terminally heat-sterilized. In addition, vitamin B12 is sensitive to the presence of other regular components of such parenteral nutrition products, including macronutrients, such as amino acids or carbohydrates, certain other vitamins and/or certain trace elements. In combination with issues around determining optimal pH and redox conditions for all components of such CB products, vitamin B12 has proved to withstand many attempts to stably include it in terminally heat-sterilized PN products as mentioned before.

Because of the above, to date there is no ready-to-use medical product for parenteral administration available that comprises a sterilized solution for parenteral administration to a patient in need thereof comprising vitamin B12 in a way that ensures stability over a prolonged period of time. In many cases, vitamin B12 must be manually added to the ready-made solutions shortly before administration or may be administered individually, and both processes are associated with a significant risk of medication error and with respect to introducing contamination to the sterilized ready-made products. Contaminations can include potential infectious agents, which represent a serious risk factor to patients in hospitals, especially those receiving nutritional solutions e.g. via parenteral administration.

In light of the prior art, there remains a significant need to develop medical products for preventing or correcting vitamin B12 deficiency in a patient that are straightforward, have a reasonable shelf-life and are easy to use, including avoiding additional mixing steps, thereby also avoiding the risk of bacterial contamination of the injectables. Due to the above-mentioned issues around the stability of vitamin B12 in solution such products are not readily available.

### SUMMARY OF THE INVENTION

The technical problem underlying the present invention is to provide a preferably terminally heat-sterilized pre-prepared and pre-packaged product for parenteral nutrition which can be used for preventing or correcting vitamin B12 deficiency in a patient comprising a ready-to-use formulation for parenteral administration provided in a flexible container comprising vitamin B12. In particular, it is the underlying problem to provide a multi-chamber bag comprising macronutrients selected from fats, carbohydrates and amino acids, peptides and proteins, and micronutrients comprising vitamins and trace elements, wherein the terminally heat-sterilized multi-chamber bag also stably comprises vitamin B12.

This problem is solved by the features of the independent claims. Preferred embodiments of the present invention are provided by the dependent claims.

The invention therefore relates to a terminally heat sterilized multi-chamber container for parenteral nutrition, comprising at least one chamber comprising an amino acid formulation, a lipid emulsion formulation or a carbohydrate formulation, and at least a second formulation comprising vitamin B12, wherein the vitamin B12 formulation
(a) is essentially free from glucose, amino acids, vitamin B1 and/or vitamin C;
(b) stably comprises vitamin B12 for at least 6 months at a temperature of from 1°C to 40°C and/or for at least 24 months at a temperature of 25°C.

According to one embodiment, the terminally heat-sterilized multi-chamber container has a reconstituted volume of more than 100 mL, such as 150 mL, 200mL, 250mL, 300mL, 400mL, 500mL, 600mL, 700mL, 800mL, 900mL, 1000mL, 1200mL, 1500mL, 1800mL, 2000mL, 2250mL, 2500mL or 3000mL. Preferably, the reconstituted volume is in a range of from 600mL to 2200 ml, especially preferably in a range of from 1200mL to 2100mL.

The expression "reconstituted volume" as used herein refers to the total volume of all solutions contained in the multi-chamber container. The expression is not intended to be limited to refer to the total volume of the solution once the seals between the chambers have been broken and the solutions contained in the respective chambers have been combined. It also refers to the added-up volume of the solutions of the separate chambers before reconstitution.

In embodiments, the vitamin B12 formulation contains no more than 1.5 ppm dissolved oxygen (DO), preferably no more than 1.0 ppm dissolved oxygen, preferably no more than 0.8 ppm dissolved oxygen and preferably no more than 0.5 ppm dissolved oxygen.

According to one embodiment of the invention, terminal heat-sterilization of the multi-chamber bag is obtained in a process with an F₀ value of at least 4 minutes, such as 4.1 minutes, 4.2 minutes or 4.3 minutes, in a process with an Fo value of at least 6 minutes, preferably at least 8 minutes.

The present invention is based on the surprising finding that it is possible to provide a terminally heat sterilized multi-chamber container for parenteral nutrition which also stably comprises vitamin B12. The multi-chamber container preferably consists of a flexible container having two or more chambers. Preferably, the multi-chamber container of the invention underwent a terminal heat-sterilization process with an Fo value of at least 8 minutes, which ensures a degree of sterility that makes the solution safe for parenteral administration. Accordingly, the present invention allows to provide a large volume product, such as the described multi-chamber container, which is terminally heat-sterilized and does not require any aseptic filling step for at least one of the comprised formulations.

In embodiments, the terminally heat sterilized multi-chamber container for parenteral nutrition has been sterilized in a way that the sterilization process has an Fo value of at least 8 minutes for all parts and components of the medical product, including the vitamin B12 formulation according to the invention. For example, in embodiments comprising more than one formulation in different chambers of the flexible container, each of the solutions is sterilized with an F₀ value of at least 8 minutes.

It was surprising that it is possible to provide a terminally heat sterilized multi-chamber container for parenteral nutrition comprising a vitamin B12 containing solution, since it has been reported in the state of the art that vitamin B12 is instable at high temperatures. It is generally accepted that vitamin B12 will be degraded upon exposure to temperatures above 100 °C, in particular when included in a complex solution comprising multiple compounds and components, for example in MCBs, and including other vitamins, nutrients and oxygen, that can interact with and destabilize vitamin B12. Specifically, it was found that vitamin B12 is basically unstable in the course of sterilization and subsequent storage in standard parenteral nutrition formulations such as, for example, lipid emulsions, glucose formulations and amino acid formulations, which makes the addition of this vitamin to parenteral nutrition products a challenge. For example, it was found that while vitamin B12 can be kept stable during sterilization in a glucose formulation, such compositions lead to a rapid degradation of vitamin B12 during subsequent storage. Accordingly, prior art solutions which provide vitamin B12 in a glucose solution were found to be undesirable.

It was also found that amino acid formulations as provided in parenteral nutrition products lead to a significant and rapid degradation of added vitamin B12 following sterilization and storage. Equally, several studies showed that irrespective of the oil source, the pH, or the stabilizing agent used, cyanocobalamin is rapidly transformed into hydroxocobalamin and that no more cyanocobalamin can be detected at T3M40°C (storage for 3 months at 40°C), even though hydroxocobalamin seems to remain relatively stable for about 6 months at 40°C.

In the experiments leading to the present invention, it was found that stability of vitamin B12 in a terminally heat sterilized multi-chamber container for parenteral nutrition can be improved when vitamin B12 is provided in an formulation which should meet certain requirements, such as oxygen levels, absence of certain compounds which seem to impact the long-term stability of vitamin B12 in said formulations, preferably has a certain pH, is light protected and undergoes sterilization at certain conditions.

For example, it is important to protect the formulation comprising vitamin B12 from light and oxygen concentrations of more than 1 ppm, preferably already during manufacture, to ensure storage stability after heat sterilization. It was also found that sterilization and storage stability can be increased at certain sterilization conditions which are defined by Fo and Co values. Also, certain pH values have been found to increase the stability of vitamin B12 in such aqueous solutions. In addition, certain stabilizing agents were found to have a positive impact on the stability of vitamin B12 during sterilization and subsequent storage, such as, for example, EDTA and/or polyvinylpyrrolidone (PVP).

Specifically, it was found that exposure of vitamin B12 to a concentration of more than 1 ppm in the process of producing the vitamin B12 formulation of the invention is detrimental for long-term stability of vitamin B12. Accordingly, in the context of the invention, exposure of the formulation and its components to a DO concentration of more than 1 ppm has to be avoided.

In the context of the invention, the concentration of DO in the vitamin B12 formulation is no more than 1 ppm. Preferably, the concentration of DO in the vitamin B12 formulation is no more than 0.5 ppm. It is particularly preferred that in the process of producing and sterilizing the formulation and preferably also in the whole manufacturing process of the medical product of the invention, it is ensured that the DO is no more than 1 ppm, preferably 0.5 ppm, especially preferably below 0.3 ppm.

In embodiments, the DO in the vitamin B12 formulation may be any value in the range of 0 to 1.5 ppm, preferably 0 to 1.0 ppm, such as for example about 0.001, 0.005, 0.01, 0.02, 0.03, 0.04, 0.05, 0.06, 0.07, 0.08, 0.09, 0.1, 0.12, 0.14, 0.16, 0.18, 0.2, 0.22, 0.24, 0.26, 0.28, 0.3, 0.32, 0.34, 0.36, 0.38, 0.4, 0.42, 0.44, 0.46, 0.48, 0.6, 0.62, 0.64, 0.66, 0.68, 0.7, 0.72, 0.74, 0.76, 0.78, 0.5, 0.52, 0.54, 0.56, 0.58, 0.8, 0.82, 0.84, 0.86, 0.88, 0.9, 0.92, 0.94, 0.96, 0.98, 0.99, 1.0, 1.1, 1.2, 1.3, 1.4 ppm.

It is a great advantage that a content of DO according to the invention is suitable for stabilizing the vitamin B12 and potentially further vitamins that can be comprised by the vitamin B12 formulation of the invention since such DO concentrations can be easily established without complicated technical instrumentation or manipulation of the solution. For example, vitamin A is equally susceptible to oxygen, and it is also susceptible to heat. In embodiments, the vitamin B12 formulation can therefore also comprise vitamin A.

Furthermore, it was found that light protection of the aqueous solution is important for reducing the loss of vitamin B12 in the formulation comprised in the MCB according to the invention. Light exposure leads to a degradation of vitamin B12 in the product during storage.

In embodiments, the vitamin B12 formulation of the terminally heat sterilized multi-chamber container for parenteral nutrition does not comprise glucose. In embodiments, the vitamin B12 formulation does not comprise amino acids. In embodiments, the vitamin B12 formulation does not comprise vitamin-Bl. In embodiments, the vitamin B12 formulation does not comprise vitamin C. In embodiments, the vitamin B12 formulation does not comprise glucose, vitamin-Bl and vitamin C.

In embodiments, the vitamin B12 formulation of the terminally heat sterilized multi-chamber container for parenteral nutrition does not comprise trace elements. In embodiments, the vitamin B12 formulation of the terminally heat sterilized multi-chamber container for parenteral nutrition does not comprise cysteine. In embodiments, the vitamin B12 formulation of the terminally heat sterilized multi-chamber container for parenteral nutrition does not comprise amino acids. In embodiments, the vitamin B12 formulation of the medical product of the invention does not comprise trace elements, cysteine and amino acids, glucose, vitamin B1 and/or vitamin C.

In embodiments, the vitamin B12 formulation of the terminally heat sterilized multi-chamber container for parenteral nutrition does not comprise trace elements, cysteine, amino acids, glucose, vitamin-B1 and vitamin C.

When testing different formulations of vitamin B12 for storage stability as part of a terminally heat-sterilized multi-chamber bag according to the invention, it was found that vitamin B12 is prone to interaction with multiple components that were considered for provision in the same solution. Importantly, it was found that inclusion trace elements into the vitamin B12 formulation is associated with destabilization of vitamin B12 and loss of vitamin B12 under the tested storage conditions.

Furthermore, it was found that inclusion of ascorbic acid (vitamin C) in the vitamin B12 formulation was associated with loss of vitamin B12. Similarly, inclusion of vitamin B1 (i.e. thiamine) in the vitamin B12 formulation was observed to contribute to vitamin B12 degradation under the tested conditions, particularly in case of terminal heat sterilization.

Importantly, when testing the option of including vitamin B12 in an aqueous solution comprising glucose, which serves as a carbohydrate source in the context of a TPN product, vitamin B12 and in particular both of cyanocobalamin and hydroxocobalamin were not stable in the solution for the required storage periods after sterilization, even though sterilization conditions can be found which provide for a relative stability of the vitamin during such sterilization.

When vitamin B12 was included in an aqueous solution intended for serving as an amino acid source of a nutritional product, it was found that the amounts of cyanocobalamin and hydroxocobalamin in the sterilized solution strongly decreased over the tested storage periods in the presence of amino acids.

In embodiments, the vitamin B12 is in the form of cyanocobalamin and/or hydroxocobalamin. Cyanocobalamin and hydroxocobalamin are the preferred vitamin B12 vitamers for inclusion in a parenteral nutritional product to prevent or treat vitamin B12 deficiency, since upon absorption they are converted into adenosylcobalamin and methylcobalamin, which are the forms which have physiological activity. Furthermore, cyanocobalamin and hydroxocobalamin are more stable in solution and are therefore advantageous for use in a sterilized ready-made nutritional product. Accordingly, the expression "vitamin B12" as used herein refers to both cyanocobalamin and hydroxocobalamin and is interchangeably used therewith, if not expressly indicated otherwise.

In embodiments of the invention, the pH of the vitamin B12 formulation is in the range of 5.0 to 9.0, preferably in the range of from 5.0 to 8.0, more preferably in the range of from 5.5 to 6.5, and especially preferably in the range of from 5.7 to 6.1. It was found that exposure to more acidic or basic environments contributes to the destabilization of vitamin B12 in its formulations.

It was unexpected that the preferred pH ranges would indeed be relevant for supporting stability of vitamin B12 formulation in the terminally heat-sterilized multi-chamber container of the invention. So far, pH had not been examined as a major parameter that influences vitamin B12 stability. In particular, the fact that especially a mildly acidic pH in the range of from 5.2 to 6.2, such as 5.9, can contribute to long-term stability of vitamin B12 was a surprising finding.

In embodiments, the pH can be any value in the claimed range. In embodiments, the pH of the vitamin B12 formulation can be about 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9 or 9.0. Preferably, the pH is about 5.5, 5.6, 5.7, 5.8, 5.9, 6.0, 6.1, 6.2, 6.3, 6.4 or 6.5., specifically about 5.7, 5.8, 5.9, 6.0 or 6.1.

In embodiments of the invention, the stability of vitamin B12 formulations according to the invention can be supported by adding stabilizing agents comprising, but not limited to, phosphate buffer, citrate buffer, EDTA, PVP and/or glutamic acid. PVP and EDTA or combinations of both have been found to be especially beneficial for further stabilizing vitamin B12 in formulation within the multi-chamber container according to the invention, and that specifically cyanocobalamin could be stabilized in the presence of EDTA and PVP, so that even the transformation of cyanocobalamin into hydroxocobalamin, which is the immediate transformation product of cyanocobalamin, could be limited.

In embodiments of the invention, the sterilized vitamin B12 formulation, as part of an MCB according to the invention, undergoes a terminal heat sterilization process that ensures a sterility corresponding to the sterility that is achieved by exposition to a sterilization temperature of 121 °C for 8 minutes. In the context of the invention, a heat sterilization process with an F₀ of at least 8 minutes is to be understood as a sterilization process that ensures a sterility corresponding to the sterility that is achieved by exposition to a sterilization temperature of 121 °C for 8 minutes. An Fo value of 8 minutes is understood as referring to 8 minutes exposition to 121°C, meaning that the solution is at a temperature of 121 °C for 8 minutes.

Accordingly, the vitamin B12 formulation of the invention may be sterilized by exposing/heating the solution to a temperature that is different from 121 °C, but the product requires to have a sterility level that corresponds to at least F₀=8 minutes in order to be considered sterile in the context of the invention.

In a preferred embodiment, the multi-chamber container for parenteral nutrition according to the invention is sterilized by moist-heat sterilization, specifically by a superheated water sterilization method. In particular, the use of superheated water sterilization methods, water cascade or water spray sterilization with a serial tower continuous sterilization equipment are preferred methods in the context of the invention, since it was found that the methods can be adjusted for applying low Fo/Co ratios to minimize the total heat-exposure of the vitamin B12 formulation, thereby reducing a loss of the vitamin during sterilization and subsequent storage..

In embodiments of the invention, the sterilization process that has been applied to the vitamin B12 formulation as part of a multi-chamber bag according to the invention has a Co value of no more than 130 minutes, preferably no more than 120 minutes, no more than 115 minutes, no more than 110 minutes, no more than 100 minutes, no more than 90 minutes, no more than 80 minutes, no more than 70 minutes, no more than 60 minutes, no more than 50 minutes and no more than 40 minutes. As used herein, the C₀ value can be understood as the time (in minutes) during which the vitamin B12 formulation is at a temperature of 100 °C or more during the sterilization process. In general, Co is a physical parameter used to quantify the total heat consumption of a sample.

In embodiments, the Co value of the sterilization process used for sterilizing the vitamin B12 formulation as part of a multi-chamber bag according to the invention can be 130, 128, 126, 124, 122, 120, 118, 116, 114, 112, 110, 108, 106, 104, 102, 100, 98, 96, 94, 92, 90, 88, 86, 84, 82, 80, 75 , 70, 65, 60, 55, 50, 45 or even 40 minutes.

In embodiments, the Fo/Co ratio of the sterilization process is not less than 0.08, more preferably not less than 0.1. In embodiments, the formulation comprising vitamin B12 underwent a sterilization process with a Fo/Co ratio of 0.08, 0.09, 0.1, 0.11, 0.12, 0.13, 0.14, 0.15, 0.16, 0.17, 0.18, 0.19, 0.2, 0.22, 0.24, 0.26, 0.28, 0.3, 0.32, 0.34, 0.36, 0.38, 0.4, 0.44, 0.48, 0.52, 0.56, 0.6, 0.65, 0.7, 0.75, 0.8, 0.9 or ideally almost 1.0.

In a further embodiment of the invention, the vitamin B12 formulation further comprises at least one B vitamin selected from the group consisting of vitamin B2 (riboflavin), vitamin B3 (niacin), vitamin B5 (pantothenic acid), vitamin B6 (pyroxidine), vitamin B8 (biotin), vitamin B9 (folate), vitamin B12 (cobalamin) and mixtures thereof.

In embodiments, the vitamin B12 formulation further comprises vitamins selected from the group consisting of vitamins B2, B3, B5, B8 and B9, preferably the solution additionally comprises vitamins B2 and B5.

In embodiments, the vitamin B12 formulation further comprises vitamin B2. In embodiments, the vitamin B12 formulation further comprises vitamin B3. In embodiments, the vitamin B12 formulation further comprises vitamin B5. In embodiments, the vitamin B12 formulation further comprises vitamin B8. In embodiments, the vitamin B12 formulation further comprises vitamin B9.

In embodiments, the vitamin B12 formulation further comprises vitamin B2 and B3. In embodiments, the vitamin B12 formulation further comprises vitamin B2 and B5. In embodiments, the vitamin B12 formulation further comprises vitamin B2 and B8. In embodiments, the vitamin B12 formulation further comprises vitamin B2 and B9. In embodiments, the vitamin B12 formulation further comprises vitamin B3 and B5. In embodiments, the vitamin B12 formulation further comprises vitamin B3 and B8. In embodiments, the vitamin B12 formulation further comprises vitamin B3 and B9. In embodiments, the vitamin B12 formulation further comprises vitamin B5 and B8. In embodiments, the vitamin B12 formulation further comprises vitamin B5 and B9. In embodiments, the vitamin B12 formulation further comprises vitamin B8 and B9.

In embodiments, the vitamin B12 formulation further comprises vitamin B2, B3 and B5. In embodiments, the vitamin B12 formulation further comprises vitamin B2, B3 and B8. In embodiments, the vitamin B12 formulation further comprises vitamin B2, B3 and B9. In embodiments, the vitamin B12 formulation further comprises vitamin B2, B5 and B8. In embodiments, the vitamin B12 formulation further comprises vitamin B2, B5 and B9. In embodiments, the vitamin B12 formulation further comprises vitamin B2, B8 and B9. In embodiments, the vitamin B12 formulation further comprises vitamin B3, B5 and B8. In embodiments, the vitamin B12 formulation further comprises vitamin B3, B5 and B9. In embodiments, the vitamin B12 formulation further comprises vitamin B3, B8 and B9. In embodiments, the vitamin B12 formulation further comprises vitamin B5, B8 and B9.

In embodiments, the vitamin B12 formulation further comprises vitamin B2, B3, B5 and B8. In embodiments, the vitamin B12 formulation further comprises vitamin B2, B3, B5 and B9. In embodiments, the vitamin B12 formulation further comprises vitamin B2, B5, B8 and B9. In embodiments, the vitamin B12 formulation further comprises vitamin B3, B5, B8 and B9. In embodiments, the vitamin B12 formulation further comprises vitamin B2, B3, B5, B8 and B9.

In embodiments, the vitamin B12 formulation constitutes or is comprised of an aqueous solution of vitamin B12 which may optionally contain further water-soluble vitamins as described herein.

In certain embodiments of the invention, the vitamin B12 formulation constitutes or is comprised by an aqueous phase of a lipid emulsion comprising an oil phase and an aqueous phase. Such embodiments are particularly preferable in case of a medical product for TPN comprising multiple containers. It is possible to include the aqueous solution comprising vitamin B12 in the same container as a lipid component, which may comprise lipid soluble vitamins. In such embodiments, the aqueous solution comprising vitamin B12 constitutes or is comprised by the aqueous phase of the lipid emulsion, wherein the lipid phase may comprise lipid soluble vitamins.

Accordingly, in different embodiments, the formulation comprising vitamin B12 can be packaged individually in a separate chamber of a multi chamber bag, for example as part of an aqueous solution or as part of a lipid emulsion; or it can be comprised by the aqueous phase a lipid emulsion.

In embodiments where the vitamin B12 is comprised in a lipid emulsion, the oil phase of the lipid emulsion may further comprise at least one fat-soluble vitamin and/or nutrient selected from the group consisting of vitamin A, retinyl palmitate, retinyl stearate, vitamin E, gamma-tocopherol, alpha-carotene, trans-beta-carotene, cis-beta-carotene, beta-cryptoxanthin, lutein, zeaxanthin, trans-lycopene, cis-lycopene, vitamin D, vitamin K and mixtures thereof. In embodiments, the lipid phase of the lipid emulsion comprising the aqueous solution which accommodates vitamin B12 may further comprise, one or more of vitamins A, D, E and K.

As used herein, the terms "oil phase" or "lipid phase" of the lipid emulsion are used interchangeably.

Accordingly, in embodiments, the multi-chamber bag of the invention comprises a lipid emulsion comprising a lipid phase and an aqueous phase comprising vitamin B12, wherein the lipid phase can comprise one or more fat-soluble vitamin as disclosed herein, preferably vitamin A, and the aqueous phase can comprise in addition to vitamin B12 further water-soluble vitamins preferably selected from B vitamins.

In embodiments, the volume of the formulation comprising vitamin B12 or of the lipid emulsion comprising vitamin B12 is in the range of 1 to 1000 ml. Any volume within this range may be used, such as 2, 3, 4, 5, 6, 7, 8, 9, 10, 12, 14, 16, 18, 20, 24, 28, 32, 36, 40, 45, 50, 55, 60, 65, 70, 75, 80, 85, 90, 95, 100, 105, 110, 115, 120, 125, 130, 135, 140, 150, 160, 170, 180, 190, 200, 220, 240, 260, 280, 300, 320, 340, 360, 380, 400, 420, 440, 460, 480, 500, 520, 540, 560, 580, 600, 620, 640, 660, 680, 700, 720, 740, 760, 780, 800, 820, 840, 860, 880, 900, 920, 940, 960, 980, 1000 ml.

In embodiments, the lipid emulsion comprising vitamin B12-is an emulsion that is different from a major lipid source of the medical product of the invention as it essentially serves only to accommodate vitamin B12 and, optionally, further vitamins. In embodiments, the medical product may therefore comprise two lipid emulsions, wherein a first lipid emulsion in a first chamber of the MCB according to the invention serves as major lipid source and can be provided in a larger volume/chamber of an MCB according to the invention, for example in the context of a ready-to-use TPN product, whereas a second lipid emulsion can comprise the vitamin B12 in a smaller volume/chamber of the MCB according to the invention . In such embodiments the second emulsion preferably has a volume in the range of about 1 to 250, more preferably about 1 to 100 ml, and especially preferably about 1 to 30 ml, while the first lipid emulsion/chamber preferably has a volume of about 40-1000 ml, more preferably 50 - 500 ml. For example, the chamber comprising the second lipid emulsion may have a volume of 10 - 50 ml, such as 15 ml, 20 ml or 25 ml, while the first lipid emulsion may have a volume in the range of 100 to 500 ml, such as 150 ml, 200ml, 300 ml or 400 ml. Such products comprising at least a first chamber and lipid emulsion and a second chamber and lipid emulsion may further comprise a third chamber comprising a carbohydrate formulation and optionally a fourth chamber comprising an amino acid formulation. The product may further comprise a fifth or sixth chamber containing additional compounds or compositions which cannot be accommodated in any of the four preceding chambers and formulations.

Vitamins C and B1 can also be comprised by the terminally heat-sterilized multi-chamber bag of the invention, but preferably these compounds are not provided in the vitamin B12 formulation.

Examples of preferable amounts of the respective vitamins that can be comprised by the medical product are listed in **Table 1.**

**Table 1. Suitable amounts and ranges of amounts of vitamins that can be used in the context of the invention. Indicated ranges are based on different ranges recommended in the art. The amounts refer to medical products providing one daily dose for one adult patient. The indicated ranges include the noted end-values. Ranges comprising any combination of disclosed end-values are considered as embodiments of the invention.**

| **Vitamin** | **Expressed as** | **Amount (Embodiment 1)** | **Amount (Embodiment 2)** | **ASPEN 2004 and 2012** | **AuSPEN 2016** |
|---|---|---|---|---|---|
| **Vitamin A (IU)** | Retinol | **3300** | 3500 | 3300 | 3500 |
| **Vitamin D3 (IU)** | Cholecalciferol | **200** | 220 | 200 | 200 |
| **Vitamin E** | α-tocopherol | **10 mg** | 11.2 IU = 10.2 mg | 10 IU | 10 mg |
| **Vitamin C (mg)** | Ascorbic acid | **200** | 125 | 200 | 110-150 |
| **Vitamin B1 (mg)** | Thiamin Chloride | **6** | 3.51 | 6 | 3 |
| **Vitamin B2 (mg)** | Riboflavin | **3.6** | 4.14 | 3.6 | 4-5 |
| **Vitamin B6 (mg)** | Pyridoxine | **6** | 4.53 | 6 | 3 |
| **Vitamin B12 (µg)** | Cyanocobalamin | **5** | 6 | 5 | 5-6 |
| **Vitamin B9 (µg)** | Folic Acid | **600** | 414 | 600 | 400 |
| **Vitamin B5 (mg)** | Panthothenic acid | **15** | 17.25 | 15 | 16-17 |
| **Vitamin B8 (µg)** | Biotin | **60** | 69 | 60 | 60 |
| **Vitamin B3/PP (mg)** | Nicotinamide | **40** | 46 | 40 | 40-47 |
| **Vitamin K (µg)** | Phytomenadione | **150** | 0 | 150 | Not required |

*ASPEN is the American Society for Parenteral and Enteral Nutrition and the indicated amounts are listed in the guidelines published in 2004 and 2012. AuSPEN is the Australasian society for parenteral and enteral nutrition and the indicated amounts are listed in the guidelines published 2016.*

In further embodiments of the invention comprising a vitamin B12 formulation which is a lipid emulsion, the lipid phase of such lipid emulsion preferably comprises or consists of a low peroxide level oil, preferably selected from the group consisting of soybean oil with low peroxides (refined soybean oil), olive oil, medium chain triglycerides, fish oil, fish oil extract, krill oil, algae oil, safflower oil, sunflower oil, corn oil, coconut oil, palm kernel oil, rapeseed oil, fungal oil and hydrogenated oils or mixtures thereof.

It was not foreseeable for a skilled person that the selection of a specific lipid components in the context of a lipid emulsion comprising vitamin B12 would be another relevant factor in enhancing stability of vitamin B12 and other vitamins present in the aqueous phase comprised by the lipid emulsion. In the process of developing the product of the invention it was found that oils or lipids with low amounts of primary and secondary oxidation products are advantageous. For example, in case soybean oil is used, it should be ensured that it comprises a low level of peroxides.

In embodiments of the invention comprising a lipid emulsion comprising vitamin B12, the lipid phase comprises or consists of a low peroxide level oil as a lipid component with a peroxide value of no more than 5 milliequivalents (mEq) O₂/kg, preferably no more than 3 mEq O₂/kg, more preferably no more than 1.5 mEq O₂/kg. Such low peroxide level oils comprise soybean oil with low peroxides (refined soybean oil), olive oil, medium chain triglycerides, fish oil, fish oil extract, krill oil, algae oil, safflower oil, sunflower oil, corn oil, coconut oil, palm kernel oil, rapeseed oil, fungal oil and hydrogenated oils or mixtures thereof.

In embodiments of the invention comprising a lipid emulsion comprising vitamin B12, the lipid phase does not comprise ascorbyl palmitate. Surprisingly, it was found that the stability of vitamin B12 is reduced in solutions comprising ascorbyl palmitate.

In embodiments of the invention, the lipid emulsion comprising vitamin B12 have a lipid concentration of 2-40 %, preferably 5-30 %, more preferably 5-20 %. In preferred embodiments, the lipid concentration is about 2-20 %, preferably 5-13%.

In embodiments, the lipid concentration of the lipid emulsion comprising vitamin B12 can be any of about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 %.

In embodiments, the vitamin B12 formulation comprises one or more stabilizing agents selected from the group comprising or consisting of
- EDTA, preferably at a concentration of no more than 0.1 g/L, and
- monobasic sodium phosphate buffer, preferably at a concentration of no more than 0.5 mM.

During the process of developing the terminally heat-sterilized multi-chamber bag of the invention, it was found that surprisingly EDTA has a stabilizing effect on vitamin B12 and specifically also cyanocobalamin. Furthermore, it was found that the use of a phosphate buffer can stabilize the pH of the vitamin B12 formulation during the sterilization process and therefore contributes to the stability of vitamin B12 in the aqueous phase of the medical product after sterilization.

In embodiments of the invention, the multi-chamber container of the invention and specifically the vitamin B12 formulation comprises from 0.5 µg to 10 µg of vitamin B12. In embodiments intended for application in adult patients, it is preferred to have amounts in the range or 2-10, 3-9, 4-8, 5-7, such as about 5 µg to provide the recommended daily dose of vitamin B12 with the product of the invention.

In embodiments of the invention for pediatric applications, the multi-chamber container of the invention and specifically the vitamin B12 formulation comprises about 0.5 - 2 µg vitamin B12, preferably about 0.7 - 1.5 µg, most preferably 0.9 µg of vitamin B12 to provide a recommended daily dose.

In embodiments of the multi-chamber container of the invention, the chamber comprising the vitamin B12 formulation has a volume of 1-250 ml, preferably 1-100 ml, more preferably 5-50 ml, most preferably 15-25 ml.

In embodiments of the invention, where the vitamin B12 formulation is comprises by a lipid emulsion, the lipid emulsion comprises an antioxidant agent, preferably selected from the group consisting of EDTA, tocopherol, and butylhydroxytoluol (BHT).

The use of an antixodant agent is particularly advantageous and indicated in embodiments where the vitamin B12 formulation is comprised by a lipid emulsion, especially where lipid components and oils are used that are prone to oxidation, such as soybean oil. Such antioxidants agents prevent or slow down autooxidation of oils and lipids/fats by giving their hydrogen to free radicals formed in the initiation and propagation stages of autoxidation. There are various kinds of antioxidants, including natural antioxidants that can be extracted from parts of olive plants, green tea, sesame, medicinal plants, etc.

In embodiments, the multi-chamber container of the invention or at least the chamber containing the vitamin B12 formulation has a light-protective outer wrapping or is made from a light protective material, respectively. This is highly advantageous since vitamin B12 is light sensitive and light protection enhances its stability in the medical product.

In embodiments of the multi-chamber container of the invention, the flexible bag comprises an oxygen barrier film, preferably with an oxygen permeability of less than 50 cc/m²/day. In further embodiments, the flexible bag comprises an inner lining containing a polyolefin, wherein the inner lining is free of polyvinyl chloride (PVC), plasticizers, adhesives or latex. In embodiments, the flexible bag comprises an oxygen barrier film, preferably with an oxygen permeability of less than 50 cc/m²/day, and an inner lining containing a polyolefin, wherein the inner lining is free of polyvinyl chloride (PVC), plasticizers, adhesives or latex.

In embodiments of the invention, the vitamin B12 is stable in the formulation and the multi-chamber container for at least three months when stored at a temperature between 1 and 50 °C.

In embodiments of the invention, the vitamin B12 is stable in the formulation and the multi-chamber container for at least 6 months, preferably 12 months, more preferably 18 months, most preferably 24 months at a temperature of from about 18°C to 32°C.

In embodiments, the formulation and the multi-chamber container as designed and prepared in the context of the present invention stabilizes vitamin B12, potentially in combination with one or more of the B vitamins listed above, in the formulation for at least three months when stored at up to 40 °C.

In further embodiments of the invention, vitamin B12 is stable in the formulation and the multi-chamber container for at least 6 months, preferably for at least 12 months, more preferably for at least 18 months, most preferably for at least 24 months.

In further embodiments of the invention, vitamin B12 is stable in the formulation and the multi-chamber container for at least 6 months, preferably 12 months, more preferably 18 months, most preferably 24 months, at a temperature of up to 30 °C.

In yet further embodiments of the invention, vitamin B12 is stable in the formulation and the multi-chamber container for at least 6 months, preferably for at least 12 months, more preferably for at least 18 months, most preferably for at least 24 months, at a temperature of from about 18°C to 25°C.

In still further embodiments of the invention, vitamin B12 is stable in the formulation and the multi-chamber container for at least 6 months, preferably for at least 12 months, more preferably for at least 18 months, most preferably for at least 24 months, at a regular storage temperature, including for example, but without limitation thereto, of temperatures of room temperature, varying from 15-30 °C, more preferably 18-25 °C, or storage in refrigerated conditions, such as from 1 to 10 °C, preferably 2 to 8, or 3 to 7 °C.

In embodiments of the invention, the multi-chamber container comprising the vitamin B12 formulation according to the invention is a sterilized solution. In some embodiments, the multi-chamber container comprising the vitamin B12 formulation according to the invention may be sterile.

It is an important advantage that the multi-chamber container according to the invention comprising the vitamin B12 formulation of the present invention can be sterilized after preparation and packaging without a significant loss of vitamin B12 and potentially further vitamins provided therein.

In preferred embodiments, the multi-chamber container comprising the vitamin B12 formulation according to the invention undergoes terminal heat sterilization after preparation and filling of the respective formulations into the container.

Sterilization is preferably done by a terminal heat sterilization process. It can also be done by using terminal filtration, gamma irradiation or any other sterilization technique. Also, an aqueous vitamin B12 formulation and further aqueous formulations as disclosed herein may be filled into the flexible bag by an aseptic filling process ensuring that no contamination of the essentially sterile emulsion occurs during filling and before sealing of the flexible bag. However, it is preferable to provide a terminally heat-sterilized product such as described herein, so as to avoid different sterilization methods during production which add to the complexity in production and carry the risk of contamination, and ultimately so a ready-to-use and safe parenteral nutrition product can be provided.

In embodiments of the invention, the terminally heat-sterilized multi-chamber container does not comprise all macronutrients selected from the group consisting of carbohydrates and proteins, and preferably the vitamin B12 formulation does not comprise any nutrients besides vitamins.

In embodiments, the terminally heat-sterilized multi-chamber container according to the invention does not comprise carbohydrates. In embodiments, it does not comprise proteins and/or amino acids. In further embodiments, the terminally heat-sterilized multi-chamber container according to the invention does not comprise electrolytes. In embodiments, the terminally heat-sterilized multi-chamber container according to the invention does not comprise trace elements.

In embodiments of the invention, the terminally heat-sterilized multi-chamber container according to the invention is configured for parenteral administration.

In embodiments of the invention, the vitamin B12 formulation is contained in one chamber of a multi chamber container having at least two, at least three, at least four, at least five or at least six chambers.

In further embodiments, the container comprises at least a first chamber containing a carbohydrate formulation, a second chamber containing a vitamin B12 formulation, a third chamber containing an amino acid formulation, a fourth chamber containing a lipid formulation, and optionally comprises electrolytes and/or vitamins in the first, third and/or fourth chamber or in a fifth chamber.

In a specific embodiment, the vitamin B12 formulation is present in a chamber comprising a lipid formulation serving as a macronutrient component of the medical product, wherein vitamin B12 is comprised by the aqueous component of such lipid emulsion. As used herein, a lipid formulation serving as a macronutrient component relates to a formulation which is intended to cover the patient's need for lipids in the context of parenteral nutrition.

In further embodiments, the container comprises at least a first chamber containing a carbohydrate formulation, a second chamber containing the vitamin B12 formulation, a third chamber containing an amino acid formulation, a fourth chamber containing a lipid formulation, and optionally comprises electrolytes and/or vitamins in the first, third and/or fourth chamber.

In embodiments, the container comprises at least a first chamber containing a carbohydrate formulation, a second chamber containing an amino acid formulation, a third chamber containing a lipid formulation, comprises electrolytes and/or vitamins in the first, second and/or third chamber and comprises vitamin B12 in a fourth chamber and optionally comprises further chambers for selected vitamins and/or trace elements, wherein the vitamin B12 formulation does not comprise any essential amounts of carbohydrates and/or amino acids, and wherein the vitamin B12 formulation is an aqueous solution or a lipid emulsion.

In a further aspect, the present invention relates to a method of preparing a terminally heat-sterilized multi-chamber container according to the invention for preventing or correcting vitamin B12 deficiency in a patient, wherein the vitamin B12 formulation is prepared by the steps comprising
a. providing an aqueous medium suitable for injection, preferably water for injection,
b. adjusting the aqueous solution to a concentration of dissolved oxygen (DO) of no more than 1 ppm and a pH value of 5-9, preferably 5 to 8,
c. dissolving vitamin B12, preferably in the form of cyanocobalamin, in the low DO solution of step b), and
d. sterilizing the solution by heat sterilization using water spray or water cascade technology.

In embodiments of the method of the invention, the vitamin B12 formulation consists of a lipid emulsion, the method comprising
a. Providing an aqueous solution suitable for injection, preferably water for injection,
b. Optionally providing a lipid phase including adding at least one fat-soluble vitamin and/or nutrient selected from the group consisting of vitamin A, retinyl palmitate, retinyl stearate, vitamin E, gamma-tocopherol, alpha-carotene, trans-beta-carotene, cis-beta-carotene, beta-cryptoxanthin, lutein, zeaxanthin, trans-lycopene, cis-lycopene, vitamin D, vitamin K and mixtures thereof to the lipid phase,
c. Separately heating up the lipid phase and the aqueous solution to a temperature of from 60°C to 90°C under agitation;
d. Preparing the pre-emulsion by transferring the lipid phase to the aqueous solution under agitation;
e. Homogenizing the pre-emulsion at a temperature of from 40°C to 60°C under pressure;
f. Optionally adding water to adjust the required volume and concentrations;
g. Adjusting the lipid emulsion to a concentration of dissolved oxygen (DO) of no more than 1 ppm and a pH value of 5-9,
h. Dissolving vitamin B12, preferably in the form of cyanocobalmin, in the low DO lipid emulsion, optionally together with additional B-vitamins, and
i. Sterilizing the solution, preferably by heat sterilization, more preferably by heat sterilization using water spray or water cascade technology.

In embodiments, the lipid phase and the aqueous phase are heated up in step c) under agitation to a temperature of 70°C - 80 °C. In further embodiments, step e) is followed by allowing the lipid emulsion to cool down to temperatures of between 15°C to 30°C, preferably room temperature.

In a further aspect, the invention relates to a terminally heat-sterilized multi-chamber container for parenteral administration for use in preventing or correcting vitamin B12 deficiency, comprising
a. vitamin B12, preferably 5-6 µg of vitamin B12, preferably in form of cyanocobalamin and hydroxocobalamin,
b. no more than 1 ppm DO, and
c. not comprising essential amounts of trace elements, glucose, amino acids, vitamint B1 and/or vitamin C.

Furthermore, the invention relates to a method of preventing or correcting vitamin B12 deficiency in a patient, the method comprising administering a formulation comprising (a) vitamin B12, preferably 5-6 µg of vitamin B12, and preferably in form of cyanocobalamin and hydroxocobalamin, (b) no more than 1 ppm DO, and (c) not comprising essential amounts of trace elements, glucose, amino acids, vitamin B1 and/or vitamin C.

In embodiments of the method of preventing or correcting vitamin B12 deficiency in a patient according to the present invention, the method comprises administering the solution reconstituted from the terminally heat-sterilized multi-chamber container for maintaining plasma levels of vitamin B12 and preventing depletion of endogenous stores in a patient receiving total parenteral nutrition.

In the context of the method of preventing or correcting vitamin B12 deficiency in a patient, the patients are adult patients. In embodiments, the patients are pediatric patients, such as neonates, pre-terms, infants, children or adolescents.

As used herein, the term "adult" refers to persons of 20 years of age and older. The term "pediatric" refers to neonates, including premature (pre-terms), full term, and post-mature neonates of up to one month of age; infants of between one month and one year of age; children of between one and up to 12 years of age, and adolescents of between 13 and up to 19 years of age.

All features disclosed in the context of the medical product for preventing or correcting vitamin B12 deficiency in a patient of the invention also relate to the method of preparing such a medical product and are herewith disclosed also in the context of the method of preparing the medical product and the other way around. The same holds true for the sterile or sterilized lipid emulsion of the invention and the method of preventing or correcting vitamin B12 deficiency in a patient according to the present invention.

### DETAILED DESCRIPTION OF THE INVENTION

All cited documents of the patent and non-patent literature are hereby incorporated by reference in their entirety.

The present invention is directed to a medical product, specifically to a terminally heat-sterilized multi-chamber container comprising a vitamin B12 formulation for preventing or correcting vitamin B12 deficiency in a patient wherein the vitamin B12 formulation does not contain more than 1 ppm dissolved oxygen (DO), and wherein the vitamin B12 formulation is essentially free from glucose, amino acids, vitamin B1 and/or vitamin C.

As disclosed herein, the terminally heat-sterilized multi-chamber container comprising a vitamin B12 formulation relates to a product intended for medical purposes, preferably for clinical nutrition. Said product of the invention is intended and designed for the medical purpose of preventing or correcting vitamin B12 deficiency in a patient, specifically for patients requiring parenteral nutrition.

The term "vitamin B12" (sometimes also referred to as "cobalamin") is a water-soluble vitamin involved in the metabolism of every cell of the human body. It is one of eight B vitamins and is a cofactor in DNA synthesis, and in both fatty acid and amino acid metabolism. It is particularly important in the normal functioning of the nervous system via its role in the synthesis of myelin, and in the maturation of developing red blood cells in the bone marrow.

The vitamin B12 exists in four near-identical chemical forms (vitamers), namely cyanocobalamin, hydroxocobalamin, adenosylcobalamin and methylcobalamin. Cobalamins are characterized by a porphyrin-like corrin nucleus that contains a single cobalt atom bound to a benzimidazolyl nucleotide and a variable residue (R) group. Because these chemical compounds have a similar molecular structure, each of which shows vitamin activity in a vitamin-deficient biological system, they are referred to as vitamers. The vitamin activity is as a coenzyme, meaning that its presence is required for some enzyme-catalyzed reactions. For cyanocobalamin, the R-residue is cyanide. For hydroxocobalamin it is a hydroxyl group. Both of these can be converted to either of the two cobalamin coenzymes that are active in human metabolism: adenosylcobalamin (AdoB12) and methylcobalamin (MeB12). AdoB12 has a 5'-deoxyadenosyl group linked to the cobalt atom at the core of the molecule; MeB12 has a methyl group at that location. These enzymatically active enzyme cofactors function, respectively, in mitochondria and cell cytosol.

Cyanocobalamin and hydroxocobalamin are used to prevent or treat vitamin deficiency; once absorbed they are converted into adenosylcobalamin and methylcobalamin, which are the forms which have physiological activity. All forms of vitamin B12 contain the biochemically rare element cobalt (chemical symbol Co) positioned in the center of a corrin ring. In the context of the present invention, the term vitamin B12 in particular refers to cyanocobalamin and hydroxocobalamin, which have been shown can be stabilized in a product according to the invention.

| | |
|---|---|
| 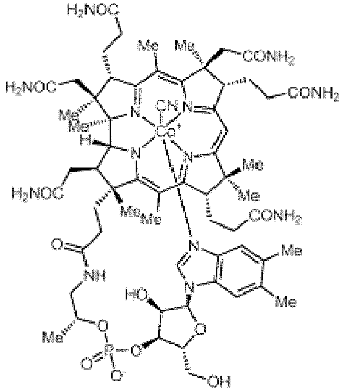 | 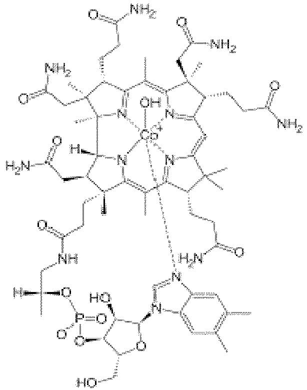 |
| **Cyanocobalamin** | **Hydroxocobalamin** |

The only organisms to produce vitamin B12 are certain bacteria, and archaea. Bacteria are found on plants that herbivores eat; they are taken into the animals' digestive system, proliferate and form part of their permanent gut flora, producing vitamin B12 internally.

Most omnivorous people in developed countries obtain enough vitamin B12 from consuming animal-sourced foods, including meat, fish, fowl, milk and eggs. Grain-based foods can be fortified by having the vitamin added to them. Vitamin B12 supplements are available as single or multivitamin tablets. Pharmaceutical preparations may be given by intramuscular injection. Because there are few non-animal sources of the vitamin, vegans are advised to consume a dietary supplement or fortified foods for B12 intake, or risk serious health consequences. Children in some regions of developing countries are at particular risk due to increased requirements during growth coupled with diets low in animal-sourced foods.

The term "vitamin B12 deficiency" relates to a condition of a patient who does not have enough vitamin B12 in his body. The most common cause of vitamin B12 deficiency in developed countries is impaired absorption due to a loss of gastric intrinsic factor, which must be bound to food-source B12 in order for absorption to occur. A second major cause is age-related decline in stomach acid production (achlorhydria), because acid exposure frees protein-bound vitamin. For the same reason, people on long-term antacid therapy, using proton-pump inhibitors, H2 blockers or other antacids are at increased risk. Deficiency may be characterized by limb neuropathy or a blood disorder called pernicious anemia, a type of megaloblastic anemia. Folate levels in the individual may affect the course of pathological changes and symptomatology of vitamin B12 deficiency. Vitamin B12 deficiency can potentially cause severe and irreversible damage, especially to the brain and nervous system. At levels only slightly lower than normal, a range of symptoms such as fatigue, lethargy, difficulty walking (staggering balance problems) depression, poor memory, breathlessness, headaches, and pale skin, among others, may be experienced, especially in people over age 60. Vitamin B12 deficiency can also cause symptoms of mania and psychosis.

The medical product of the invention can be used in the treatment of patients having, developing or being at risk of developing vitamin B12 deficiency. The treatment can aim to prevent or correct a vitamin B12 deficiency in this patient group.

Furthermore, in embodiments the medical product of the invention may be used as TPN product comprising most or preferably practically all necessary nutrients to ensure the entire nutrient supply of a patient. Accordingly, in such embodiments the product is intended to prevent vitamin B12 deficiency of a patient who is not consuming other nutrients than those provided by the medical product of the invention.

In the context of the invention, the product of the invention comprises a terminally heat-sterilized, flexible multi-chamber container which is designed to accommodate at least two formulations, preferably made from an oxygen-impermeable material, which comprises a vitamin B12 formulation wherein vitamin B12 remains stable for at least 6 months at a temperature of from 1°C to 40°C. In the context of the invention, an example of an oxygen-impermeable material is an oxygen barrier film, preferably with an oxygen permeability of less than 50 cc/m²/day.

The term "stable" as used herein in connection with vitamin B12 means that at least 50%, at least 60%, at least 70% or at least 80% of the amount of vitamin B12 initially provided in the product is still available after terminal heat-sterilization and storage of the terminally heat-sterilized product of the invention for at least 6 months at a temperature of from 1°C to 40°C and/or for at least 24 months at temperatures of from 1°C to 25°C. Preferably, at least 80%, at least 85%, at least 90% and at least 95% of the vitamin B12 provided in the product at the time it is produced is still available after terminal heat-sterilization and storage of the terminally heat-sterilized product of the invention for at least 6 months at a temperature of from 1°C to 40°C and/or for 24 months at a temperature from 1°C to 25°C. Generally, it will be assumed that the amount of vitamin B12 "provided in the product" corresponds to the amount stated in a product description. As mentioned before, the term "vitamin B12", if not indicated otherwise, refers to cyanocobalamin and hydroxocobalamin.

The term "dissolved oxygen" (DO) as used herein refers to the level of free, non-compound oxygen present in liquids, such as water, lipid emulsions or other liquids or solutions. Oxygen saturation (symbol SO₂) is a relative measure of the concentration of oxygen that is dissolved or carried in a given medium as a proportion of the maximal concentration that can be dissolved in that medium. It can be measured with a dissolved oxygen probe such as an oxygen sensor or an optode in liquid media, usually water.

Dissolved oxygen is usually reported in milligrams per liter (mg/L) or as a percent of air saturation. However, studies also report DO in parts per million (ppm) or in micromoles (pmol). 1 mg/L is equal to 1 ppm. The relationship between mg/L and % air saturation varies with temperature, pressure and salinity of the water. One micromole of oxygen is equal to 0.022391 milligrams. Thus 100 µmol/L O₂ is equal to 2.2 mg/L O₂. To calculate dissolved oxygen concentrations from air saturation, it is necessary to know the temperature and salinity of the sample. Barometric pressure has already been accounted for as the partial pressure of oxygen contributes to the percent air saturation. Salinity and temperature can then be used in Henry's Law to calculate what the DO concentration would be at 100% air saturation. However, it is easier to use an oxygen solubility chart. These charts show the dissolved oxygen concentration at 100% air saturation at varying temperatures, and salinities. This value can then be multiplied by the measured percent air saturation to calculate the dissolved oxygen concentration [Fondriest Environmental, Inc. "Dissolved Oxygen." Fundamentals of Environmental Measurements. 19 Nov. 2013.].

Oxygenation of liquids occurs for example through exposure of the liquids to gases containing oxygen. For example, exposure of a liquid sample to the atmosphere comprising around 21% O₂ leads to oxygenation through diffusion of the gaseous oxygen into the liquid. This process can be accelerated for example by stirring, flushing the liquid with an oxygen-containing gas or similar techniques known to the skilled person.

There are several methods available in the art for measuring dissolved oxygen concentrations. Modern techniques involve either an electrochemical or optical sensor, where the dissolved oxygen sensor is attached to a meter for spot sampling and laboratory applications or to a data logger, process monitor or transmitter for deployed measurements and process control. An example is the fiber-optic oxygen meter Microx TX3 from PreSens Precision Sensing GmbH (Germany) for gaseous and dissolved O₂. The colorimetric method offers a basic approximation of dissolved oxygen concentrations in a sample. There are two methods designed for high-range and low-range dissolved oxygen concentrations. These methods are quick and inexpensive for basic projects but limited in scope and subject to error due to other redoxing agents that may be present in the water. The traditional method is the Winkler titration.

Methods of deoxygenation are known to a person skilled in the art, including for example gas-flushing with suitable gases, such as nitrogen, or by use of vacuum deaerators.

In embodiments of the invention, the product according to the invention comprising vitamin B12 is a sterilized product. In the context of the invention, the term "sterilized" relates to a product, solution or emulsion that has undergone a process of sterilization. Sterilization refers to any process that eliminates, removes, kills, or deactivates all forms of life (in particular referring to microorganisms such as fungi, bacteria, viruses, spores, unicellular eukaryotic organisms such as Plasmodium, etc.) and other biological agents like prions present in a specific surface, object or fluid, for example food or biological culture media. Sterilization can be achieved through various means, including heat, chemicals, irradiation, high pressure, and filtration. Sterilization is distinct from disinfection, sanitization, and pasteurization, in that those methods reduce rather than eliminate all forms of life and biological agents present. After sterilization, an object is referred to as being sterile or aseptic.

The product according to the invention comprising vitamin B12 is terminally heat-sterilized. Terminal heat sterilization is the preferred method for drug products, including parenteral nutrition products, because in this process sterilization takes place after the product has been filled into the primary packaging. Because of this, there are no further opportunities for contamination due to human intervention. Terminally sterilized products are treated in a microbially lethal process and hence represent the lowest risk category of sterile pharmaceutical products unlike products aseptically manufactured in a microbiologically controlled environment.

In embodiments employing moist-heat sterilization, sterilization involves heating under pressure in the presence of water to generate steam; this method is recommended by various pharmacopeias. Generally, said steam sterilization is performed in an autoclave and can be used for drug products, medical devices, plastic bags and other single-use equipment, glass containers, surgical dressings and more. Terminal sterilization with moist heat (i.e., steam) is typically recommended with heating to 121 °C at 15 psi.

The expression "terminally sterilized" or "terminally heat-sterilized" means that such products must have a probability of non-sterile unit (PNSU) or a sterility assurance level (SAL) of not more than one in a million units produced, in accordance with the guidelines in Europe and the United States. SAL has been defined by European Pharmacopoeia in such a way that its numerical value is the same of PNSU. Accordingly, a SAL or PNSU of 10⁻⁶ indicates that the probability of an organism surviving to the end of the sterilization process in any single unit of product is less than one in one million. The proof that a terminally sterilized product complies with the 10⁻⁶ SAL/PNSU can be accomplished by several different sterilization cycle development approaches. The proper application of this method requires extensive scientific knowledge regarding the sterilization method selected for use with a specific product. Further background information is provided, for example, in von Woedtke and Kramer, GMS KHI (2008), 3(3), 1-10 (ISSN 1863-5245). The expression "sterility" means the absence of all viable microorganisms including viruses.

In the context of the invention, the multi-chamber container comprising vitamin B12 can be terminally sterilized by superheated water sterilization methods. Such methods include, for example, water cascade sterilization and water spray sterilization, including methods employing serial tower continuous sterilization equipment. Superheated water is liquid water under pressure at temperatures between the usual boiling point, 100 °C (212 °F) and the critical temperature, 374 °C (705 °F). It is also known as "subcritical water" or "pressurized hot water."

Superheated water is stable because of overpressure that raises the boiling point, or by heating it in a sealed vessel with a headspace, where the liquid water is in equilibrium with vapor at the saturated vapor pressure.

Superheated water spray sterilizers are designed to meet terminal sterilization requirements in pharmaceutical industry, based on the principle of moist-heat sterilization under counter pressure. These superheated Sterilizers are fitted with water recirculation through sanitary pump and spray system to heat up and cool down the products to be sterilized. These units are ideal for infusion products, blister packed products and large volumes of liquids sealed in glass or plastic containers. In certain superheated water spray sterilizers, The product is introduced in the sterilizer chamber on appropriate carriages and trays. The chamber bottom is filled with purified water which contains low bio-burden. This water is circulated in a closed loop, utilizing a high-quality centrifugal pump. The water passes through an external heat exchanger to reach appropriate sterilization temperature before it is sprayed through a perforated distribution plate from the top of the chamber in the cascade form. The elevated flow rate ensures even temperature throughout the chamber during the sterilization process. The water reaches the sterilization temperature quickly. The counter pressure is applied using the sterile filtered air to prevent any damage to plastic container or rubber closures from being displaced from glass bottles. After the sterilization cycle is over, the water is indirectly cooled through the external heat exchanger. The same cooled water remains in circulation resulting in cooling of load in the chamber.

Superheated water cascade systems are also very useful for terminally sterilizing the product of the invention. Such systems enable liquids in closed receptacles made of glass or other temperature-resistant materials (such as flexible bags used in the context of the present invention) to be sterilized quickly, reliably and gently. The advantage of the hot water cascade system lies in its very short cycle times, which are achieved through a high circulation rate and cascade density in combination with short heating up and cooling down times.

In certain water cascade systems, the chamber containing the item to be sterilized is filled to a predefined level (below the item being sterilized) with deionized sterilization water. This water then circulates through a steam-heated heat exchanger and cascades over the item being sterilized at a continuously rising temperature. This efficient method of transferring heat enables the item to be heated up in a quick and gentle way. In the subsequent cooling phase, the sterilization water flows through the now water-cooled heat exchanger and cools the item being sterilized down to a specific temperature. Throughout the process, temperature-controlled supporting pressure generated by sterile-filtered compressed air prevents the tightly closed receptacle from bursting or deforming. Liquids are usually sterilized at 121 °C for a time that is adjusted depending on product requirements.

Sterilization can also be achieved via dry heating. However, much higher temperatures (180-200 °C) are required for this method, which is unfavorable for heat-sensitive products such as described herein. Dry heating is commonly used to sterilize glassware, metal and other surfaces.

In embodiments of the invention, sterilization of the multi-chamber container and the vitamin B12 formulation contained therein is performed in a terminal heat-sterilization process with an F₀ value of at least 8 minutes. In embodiments, the F₀ value of the sterilization process is at least 8 minutes. In embodiments, the F₀ value of the terminal heat sterilization process is 8.5, 9, 9.5, 10, 11, 12, 13, 14, 15, 16, 17, 18, 19, 20, 21, 22, 23, 24, 25, 26, 27, 28, 29, 30, 32, 34, 36, 38, 40, 42, 44, 46, 48, 50 minutes.

In embodiments of the invention, the terminal heat sterilization of the product according to the invention has a C₀ value of no more than 130 minutes, preferably no more than 125, 120, 115, 110, 105, 100, 95, 90, 85, 80, 75, 70, 65, 60, 55, 50, 45 or 40 minutes, especially preferably no more than 115, 110, 105, 100, 95, 90, 85, 80, 75, 70, 65 or 60 minutes.

C₀ and F₀ are two quantitative parameters used to evaluate the amount of heat transferred to a product or solution during a moist-heat sterilization process. F₀ describes the lethality performance of the process whereas C₀ relates to the *chemical degradation of the API.* The ratio of F₀ vs. C₀ (F₀/C₀) is important to control in order to avoid too much heat degradation at a defined lethality level.

F₀ and C₀ can be calculated by using the following formulas:

| F₀ | C₀ |
|---|---|
| F₀ = *∫* 10^((T - 121) /Z₁₂₁) dt | C₀ = *∫* 10^((T - 100)/Z_{C}) dt |
| Z_{121°c} = 10°C | Z_{C} = 33°C |

In certain standard protocols for heat sterilization, sterilization is done at 121 °C, which can be considered a standard temperature for sterilization. If a material is sterilized at a temperature other than 121 °C the time of sterilization leading to the same result of killing microorganisms changes. Some materials are heat sensitive and cannot be sterilized at 121 °C. The F₀ value is used to determine the exposure time of a material for sterilization at a particular temperature.

By calculating the F₀ value, the required sterilization time can be minimized. The F₀ value indicates the sterilization time at a constant temperature. This means that the F₀ value can be expressed as sterilization work within a specific sterilization process and is expressed in minutes with reference to a specific temperature. For a given F₀ value, which is given as a reference for many industrial processes at a sterilization temperature of 121°C and a z-value of 10°C, the sterilization time can be calculated to achieve a certain final bacterial count. As used herein, the F₀ refers to a sterilization temperature of 121 °C and a z-value of 10 °C. See also Pistolesi and Mascherapa, F₀ A technical note (1988, revised 2014), Issued by R&D Fedegari.

In a real process, the sterilizer, such as an autoclave or water cascade sterilizer, heats the product for a certain period of time until the sterilization temperature of 121°C is reached. During the heating time of 100°C - 121°C and also during the cooling time, microorganisms are killed. Taking this killing effect during the heating and cooling time into account, the holding time can be shortened accordingly. Thus, the desired F₀ value can be achieved with a shorter overall process time. A prerequisite is the previous determination of the "coldest or most difficult to sterilize point" in the load. An F₀ value of 1 minute is the heat effect (killing effect) of 121.1 °C within one minute.

In other words, sterilization by heating involves a heating and a cooling phase, during which lethal temperatures are also passed through, but these are not as effective as the higher temperature of the holding phase. These phases also contribute to the sterilization effect. In order to have a simple measure for the overall effect, the F-value was introduced. It is a measure of the sum of all killing effects during the entire heating process, including the heating, holding and cooling phases, expressed as time equivalent for a reference temperature. The killing effects are lower in the heating and cooling phase than in the holding phase because of the lower temperatures, depending on the z-value which is characteristic for the respective microorganisms. In practice, 121.1 °C is chosen as the reference temperature and 10 as the z-value and the resulting value is called the Fo-value. It is given in minutes. An Fo value of 8 means that the entire heating process has the same killing effect on microorganisms with a z value of 10 as heating for 8 minutes at 121.1 °C.

As defined above, the term F-value or "F_{Tref/z}" is defined as the equivalent number of minutes to a certain reference temperature (Tref) for a certain control microorganism with an established Z-value. Z-value is a term used in microbial thermal death time calculations. It is the number of degrees the temperature has to be increased to achieve a tenfold (i.e. 1 log10) reduction in the D-value. The D-value of an organism is the time required in a given medium, at a given temperature, for a ten-fold reduction in the number of organisms. It is useful when examining the effectiveness of thermal inactivations under different conditions. The z-value of an organism in a particular medium is the temperature change required for the D-value to change by a factor of ten, or put another way, the temperature required for the thermal destruction curve to move one log cycle. It is the reciprocal of the slope resulting from the plot of the logarithm of the D-value versus the temperature at which the D-value was obtained. While the D-value gives the time needed at a certain temperature to kill 90% of the organisms, the z-value relates the resistance of an organism to differing temperatures. The z-value allows calculation of the equivalency of two thermal processes, if the D-value and the z-value are known.

Exposure to radiation is another sterilization method used throughout the industry. Gamma radiation is the most common, though other options include infrared and ultraviolet radiation and high-velocity electrons. Radiation is typically used for the sterilization of single-use components/systems, but it can also be used for packaged drug products.

Treatment with gases is also a sterilization alternative. Such gases include ethylene oxide, formaldehyde, glutaraldehyde, propylene oxide, hydrogen peroxide and chlorine dioxide. This method is more commonly used to sterilize clean-room suites. Sterilization via filtration is the only option if the other processes are not suitable for the specific product or component. In filtration, the final drug product solution is passed through a filter that has been produced under aseptic manufacturing conditions and designed with appropriate pore sizes/surface chemistries that remove bacteria via size exclusion, entrapment, electrostatic attraction and other modalities.

As used herein, a "nutrient" is a substance used by an organism, such as a human, to survive, grow, and reproduce. Nutrients can be incorporated into cells for metabolic purposes or excreted by cells to create non-cellular structures, such as hair, scales, feathers, or exoskeletons. Some nutrients can be metabolically converted to smaller molecules in the process of releasing energy, such as for carbohydrates, lipids, proteins/amino acids, and fermentation products (ethanol or vinegar), leading to end-products of water and carbon dioxide. All organisms require water. Essential nutrients for animals and humans are the energy sources, some of the amino acids that are combined to create proteins, a subset of fatty acids, vitamins and certain minerals/trace elements.

A classification used primarily to describe nutrient needs of animals divides nutrients into "macronutrients" and "micronutrients". Consumed in relatively large amounts, macronutrients (carbohydrates, lipids/fats, proteins/amino acids, water) are used primarily to generate energy or to incorporate into tissues for growth and repair. Micronutrients are needed in smaller amounts; they have subtle biochemical and physiological roles in cellular processes, like vascular functions or nerve conduction. Inadequate amounts of essential nutrients, or diseases that interfere with absorption, result in a deficiency state that compromises growth, survival and reproduction.

Macronutrients comprise carbohydrates, proteins, lipids, and water. Macronutrients are defined as a class of chemical compounds which humans consume in the largest quantities and which provide humans with the bulk of energy. While water does make up a large proportion of the total mass ingested as part of a normal diet, it does not provide any nutritional value. Carbohydrates comprise glucose, sucrose, ribose, amylose (a major component of starch), amylopectin, maltose, galactose, fructose, lactose. Proteins are composed of amino acids comprising standard amino acids alanine, arginine, aspartic acid (aspartate), asparagine, cysteine, glutamic acid (glutamate), glutamine, glycine, histidine, isoleucine (branched chain amino acid), leucine (branched chain amino acid), lysine, methionine, phenylalanine, proline, serine, threonine, tryptophan, tyrosine, valine (branched chain amino acid). Lipids (or fats) comprise saturated fats such as butyric acid (C4), caproic acid (C6), caprylic acid (C8), capric acid (C10), lauric acid (C12), myristic acid (C14), pentadecanoic acid (C15), palmitic acid (C16), margaric acid (C17), stearic acid (C18), arachidic acid (C20), behenic acid (C22), lignoceric acid (C24), cerotic acid (C26); monounsaturated fats such as myristol, pentadecanoic, palmitoyl, heptadecenoic, oleic acid, eicosen, erucic acid, nervonic acid; polyunsaturated fats such as linoleic acid (LA) - an essential fatty acid, α-Linolenic acid (ALA) - an essential fatty acid, stearidonic acid (SDA), arachidonic acid (ETA), timnodonic acid (EPA), clupanodonic acid (DPA), cervonic acid (DHA); essential fatty acids, such as α-Linolenic acid ALA (18:3) Omega-3 fatty acid and Linoleic acid LA (18:2) Omega-6 fatty acid, being the starting point for other important omega-acids (e.g. DHA, EPA) .

Micronutrients are essential elements required by human in small quantities throughout life to orchestrate a range of physiological functions to maintain health, in particular vitamins and dietary minerals/trace elements. Trace elements/trace minerals/dietary minerals include Boron, Cobalt (as a component of vitamin B12), Fluoride, Chromium, Copper, Iodine, Iron, Manganese, Molybdenum, Selenium and Zinc.

In the context of the present invention, trace elements may be provided, for example, as chloride or sodium salts, such as zinc chloride, iron chloride, copper chloride, sodium selenite, manganese chloride, sodium fluoride, potassium iodide, chromium chloride, sodium molybdate.

Vitamins comprise Vitamin B complex, Vitamin B1 (thiamin), Vitamin B2 (riboflavin), Vitamin B3 (niacin), Vitamin B5 (pantothenic acid), Vitamin B6 group (Pyridoxine, Pyridoxal-5-Phosphate, Pyridoxamine), Vitamin B8 (biotin), Vitamin B9 (folate), Vitamin B12 (cobalamin), Choline, Vitamin A (e.g. retinol (see also - provitamin A carotenoids)), Vitamin C (Ascorbic acid), Vitamin D (vitamin D2 (Ergocalciferol), vitamin D3 (Cholecalciferol)), Vitamin E (tocopherols and tocotrienols), Vitamin K (Vitamin K1 (phylloquinone), Vitamin K2 (menaquinone)). For the avoidance of doubt, the expression "vitamin B8" (biotin) as referred to herein can be used interchangeably with the expression "vitamin B7".

The multi-chamber container of the present invention in embodiments can comprise the fat-soluble vitamins A, D, E and/or K. According to one embodiment of the invention, the multi-chamber container of the invention comprises vitamins A, D, E and K.

In the context of the invention, the use of retinyl palmitate (formula C₃₆H₆₀O₂) as a vitamin A source, also referred to as vitamin A palmitate or retinol palmitate, and which is the ester of retinol and palmitic acid, is particularly preferred, since it was found that this form of vitamin A is associated with a very good stability in the context of the medical product of the invention.

As used herein, the term vitamin D relates to a group of fat-soluble secosteroids responsible for increasing intestinal absorption of calcium, magnesium, and phosphate, and multiple other biological effects. In humans, the most important compounds in this group are vitamin D3 (also known as cholecalciferol) and vitamin D2 (ergocalciferol).

As used herein, vitamin E is a group of eight fat soluble compounds that include four tocopherols and four tocotrienols. Both the tocopherols and tocotrienols occur in α (alpha), β (beta), γ (gamma) and δ (delta) forms, as determined by the number and position of methyl groups on the chromanol ring. All eight of these vitamers feature a chromane double ring, with a hydroxyl group that can donate a hydrogen atom to reduce free radicals, and a hydrophobic side chain which allows for penetration into biological membranes. Of the many different forms of vitamin E, gamma-tocopherol (γ-tocopherol) is the most common form found in the North American diet, but alpha-tocopherol (α-tocopherol) is the most biologically active. All forms of vitamin E can be comprised by the medical product of the invention.

As used herein, the term vitamin K relates to a group of structurally similar, fat-soluble vitamins found in foods and in dietary supplements. The human body requires vitamin K for complete synthesis of certain proteins that are needed for blood coagulation or for controlling binding of calcium in bones and other tissues. The complete synthesis involves final modification of these so-called "gla-proteins" by the enzyme gamma-glutamyl carboxylase that uses vitamin K as a cofactor. This modification allows them to bind (chelate) calcium ions, which they cannot do otherwise.

Electrolytes, such as sodium, potassium, chloride, calcium, magnesium, phosphate and bicarbonate may also be classified as nutrients.

Besides the vitamin B12 formulations comprised of a lipid emulsion and optionally comprising vitamins A, D, E and K, the vitamin B12 formulation of the invention may comprise further nutrients, such as selected macronutrients and micronutrients. The respective formulations of the terminally heat-sterilized multi-chamber container may be completely or partially reconstituted before administration to a patient. Administration of the one or more formulations of the product, generally after complete or partial reconstitution of the two or more formulations, can occur through various routes of administration. Generally, the product of the invention is administered parenterally. Generally, the product will be administered by central parenteral nutrition (CPN) or, depending on the specific formulation in terms of, for example, osmolality, pH and infusion rate, by peripheral parenteral nutrition (PPN). In the context of the present invention, CPN will generally be preferred, especially in cases where lipid emulsions are comprised. However, PPN is also an option for certain embodiments of the invention.

As used herein, "reconstituted solution" refers to a solution for parenteral administration, which is generated by admixing the content of the chambers of a multi-chamber container before use.

Parenteral administration is preferred in the context of the invention. Parenteral nutrition (PN) is the feeding of specialist nutritional products to a person intravenously, bypassing the usual process of eating and digestion. It is called total parenteral nutrition (TPN) or total nutrient admixture (TNA) when no significant nutrition is obtained by other routes, and partial parenteral nutrition (PPN) when nutrition is also partially enteric. It may be called peripheral parenteral nutrition (PPN) when administered through vein access in a limb rather than through a central vein as central venous nutrition (CVN). Enteral food administration is via the human gastrointestinal tract and contrasts with parenteral administration.

In the context of the medical product of the invention, the lipid emulsion is provided in a flexible container. In embodiments of the invention, the lipid emulsion is contained in one chamber of a multi chamber container having at least two, at least three, at least four, at least five or at least six chambers.

As used herein, the term "flexible container" refers to a container or bag made of a flexible material, such as bags made from plastic films. The term does not encompass polymeric rigid or semirigid containers.

Flexible containers or bags of the invention can be made of materials comprising, without limitation, polyvinyl chloride (PVC), polypropylene (PP), polyethylene (PE), ethylene vinyl alcohol (EVOH), ethylene-vinyl acetate (EVA) and all possible copolymers, essentially any synthetic material suitable for containing the components to be administered.

Oxygen impermeable flexible containers are made of gas barrier films that block oxygen migration to the outside of the container. Different technologies have been developed in order to provide oxygen barrier to transparent films, such as PE films or polyethylene terephthalate films. The main technologies are the following: (1) Coating with high barrier materials, generally inorganic oxide layers (e.g. SiOx or Al₂O₃); (2) Multilayer films, wherein an inner layer consists a barrier material such as EVOH, polyamide, aluminum, halogenated polyvinylidene such as PVDC, amorphous nylon or crystalline nylon or combination of both, copolymers of ethylene vinyl alcohol copolymer layer (EVOH), polyolefins, including combinations of two or more of the above layers, and wherein the outer layers consist of structural polymer (e.g. PE, PP or PET).

The disclosure therefore also provides for a flexible container, preferably a multi-chamber container for parenteral or nutritional formulations which may be prepared from any of the beforementioned flexible films. For example, the container may be in the form of a bag having one or multiple compartments or chambers. The container, such as a bag, may include at least two chambers, but may also contain three, four, five or six or more chambers, and in one preferred embodiment, two or three chambers.

Suitable containers typically are sterile, non-pyrogenic, single-use, and/or ready-to-use. The multi-chamber containers are particularly useful for holding a parenteral nutrition product for adults, children or neonates and can provide a carbohydrate formulation as disclosed herein in the first chamber, an amino acid formulation as disclosed herein in a second chamber, and a lipid formulation as disclosed herein in a third chamber of the container.

The multi-chamber container may include vertical chambers as disclosed in U.S. Patent Publication No. 2007/0092579. For example, the multi-chamber container may be configured as a bag that includes two, three, four, five or six adjacent chambers or compartments. If desired, frangible barriers or openable seals (e.g., peel seals or frangible seals) are used to separate the chambers of the multi-chamber container. Multi-chamber containers may also comprise three chambers for accommodating a lipid emulsion, a carbohydrate formulation and an amino acid formulation, and further comprise at least one, in certain embodiments two or three smaller chambers which contain, for example, vitamin formulations and/or trace element formulations. In one specific embodiment, the multi-chamber container of the invention has a first chamber containing the lipid emulsion according to the invention, a second chamber containing an amino acid formulation, a third chamber containing a carbohydrate formulation, a fourth chamber containing a vitamin formulation and a fifth chamber containing a trace element formulation.

A multi chamber container or multi chamber bag (MCB) as used in the context of the invention may be designed for the parenteral administration of its reconstituted content after admixing the formulations contained in the respective chambers. Such MCB may have 2, 3, 4, 5, 6 or more chambers. The chambers of said MCB may have the same size or may have different sizes to accommodate various compositions and volumes. The chambers may be designed to contain volumes of from, for example, 1 to 5 ml, from 5 to 10 ml, from 10 to 50 ml, from 50 to 100 ml, from 100 to 250 ml, from 250 ml to 500 ml, from 500 to 1000 ml, or from 1000 to 1500 ml. The MCBs can be designed to have chambers which are located adjacent to each other. The chambers may have various shapes. The chambers can be oriented horizontally and/or vertically to each other. Certain small chambers can be designed to be located within another, larger chamber, wherein, for example, the small chamber which is located within another, larger chamber can be accommodated and fixed into said larger chamber by welding at least one edge of said small chamber in between the weld seam of the surrounding larger chamber.

The openable seals of said multi-chamber containers permit formulations to be separately stored and admixed/reconstituted just prior to administration thereby allowing storage in a single container of formulations which should not be stored as an admixture for an extended period of time. Opening of the seals allows communication between the chambers and mixing of the contents of the respective chambers. The outside seals of the multi-chamber container are strong seals that do not open under the fluid pressure supplied to open the weaker peel seals or frangible seals between the chambers. In some embodiments, the openable seals of the multi-chamber container may be designed to allow for the admixing or reconstitution of only selected chambers of the multi-chamber container, for example, the admixing of the lipid emulsion with the vitamin chamber and the amino acid chamber, if so desired.

The multi-chamber container may be provided with instructions explaining a desired order with which to open the peel seals, so that constituent fluids are mixed in a desired order. The unsealing strengths of the two or more peel seals may be varied to promote the opening of the seals in the desired order. For example, the unsealing strength of the peel seal to be opened first may be 1/3 to 1/2 of the unsealing strength required to open the peel seal to be opened second.

As used herein, amino acid formulations include a sterile, aqueous solution of one or more amino acids and one or more electrolytes. Typically, amino acid formulations include about 2 g to about 20 grams of amino acids per 100 mL of amino acid formulation, such as about 4 grams to about 15 grams per 100 mL of amino acid formulation. Typical amino acids which are included into amino acid formulations are, for example, isoleucine, leucine, valine, lysine, methionine, phenylalanine, threonine, tryptophan, arginine, histidine, alanine, aspartic acid, cysteine, glutamic acid, glycine, proline, serine, tyrosine, ornithine, and taurine.

The amino acid formulation may further include electrolytes. As used herein, electrolytes include sodium, potassium, calcium, magnesium, and/or phosphate ions. For example, the amino acid formulation can include from about 0.1 mmol to about 10 mmol of sodium (e.g., about 3.75 mmol to about 10 mmol of sodium), from about 0.1 mmol to about 10 mmol of potassium (e.g., about 3.75 mmol to about 6.90 mmol of potassium), from about 0.05 mmol to about 1.0 mmol of magnesium (e.g., about 0.05 mmol to about 0.11 mmol and/or about 0.38 mmol to about 0.65 mmol of magnesium), from about 0.1 mmol to about 10 mmol of calcium (e.g., about 1.13 mmol to about 5.10 mmol of calcium), from about 0.1 mmol to about 10 mmol of phosphate (e.g., about 0.94 mmol to about 5.10 mmol of phosphate) and not more than 10 mmol of chloride (e.g., not more than 5.6 mmol of chloride) per 100 mL of amino acid formulation. When calcium and phosphorus are present together in the same heat-sterilized solution, insoluble calcium phosphate precipitation can occur. So, calcium may be omitted from the amino acid formulation. Using an organic salt of phosphorus such as sodium glycerophosphate 5·H₂O or calcium glycerophosphate, calcium and phosphate amounts may be increased without solubility issues and without providing excess sodium or chloride. In the amino acid formulation, sodium may be provided in the form of sodium chloride, magnesium may be provided in the form of magnesium acetate 4·H2O or magnesium chloride, and potassium may be provided in the form of potassium acetate.

Carbohydrate formulations provide a supply of calories, typically in the form of glucose. In particular, the carbohydrate formulation provides an amount of carbohydrate sufficient to avoid adverse effects such as hyperglycemia that has been observed in patients receiving parenteral nutrition. Typically, the carbohydrate formulation includes about 20 to 75 grams of glucose per 100 mL of carbohydrate formulation, such as, for example, from 20 to 60 grams or from 25 to 55 grams of glucose per 100 mL of carbohydrate formulation. Carbohydrate formulations may further contain calcium in an amount of from 0.1 to 10 mmol/100 ml of the carbohydrate solution, such as, for example, from 1.0 to 5.0 mmol/100 ml or from 1.0 to 2.0 mmol/100 ml.

Lipid formulations that serve as a source of lipids as a macronutrient component of the medical product of the invention are an emulsion of an oil phase, a water phase, and an emulsifier that makes the two phases miscible. Furthermore, the medical product comprises a lipid emulsion as claimed herein that comprises vitamin A, D, E and K. The lipid emulsion comprising vitamin A, D, E and K can be comprised by the lipid formulation serving as a major lipid source, or can be a different liquid in a different chamber of the medical product.

In the context of the invention, in case of lipid emulsions, which are to be used as an injectable emulsion for parenteral nutrition, the emulsion must be an oil-in-water (o/w) emulsion. This means that the oil must reside in the internal (or dispersed) phase, while water is the external (or continuous) phase, as the emulsion must be miscible with blood. Lipid emulsion as disclosed herein must therefore also be substantially free of any suspended solids. Of course, the lipid emulsions may contain further components, including, but not limited to, antioxidants, pH modifiers, isotonic agents, vitamins, trace elements and various combinations thereof. An overview over lipid emulsions, their composition and use is provided, for example, in Driscoll, Journal of Parenteral and Enteral Nutrition 2017, 41, 125-134. Further information on the use of lipid emulsions in parenteral nutrition of intensive care patients is provided, for example, in Calder et al, Intensive Care Medicine, 2010, 36(5), 735-749.

The oil phase of the lipid emulsion may include polyunsaturated fatty acids, such as long-chain polyunsaturated fatty acids, which may be present as the free acid, as an ionized or salt form of the free acid, and/or in ester form. Suitable esters of the polyunsaturated fatty acids/long-chain polyunsaturated fatty acids include, but are not limited to, alkyl esters (e.g., methyl esters, ethyl esters, propyl esters, or combinations thereof) and triglyceride esters. In some cases, the long-chain polyunsaturated fatty acid has a structure R(C=O)OR', wherein R is an alkenyl group having at least 17 carbon atoms, at least 19 carbon atoms, at least 21 carbon atoms, or at least 23 carbon atoms, and R' is absent, H, a counter ion, an alkyl group (e.g., methyl, ethyl, or propyl), or a glyceryl group (e.g., R(C=O)OR' is a monoglyceride, a diglyceride, or a triglyceride). Polyunsaturated fatty acids for use in the lipid formulations disclosed herein include, but are not limited to, linoleic acid (LA), arachidonic acid (ARA), α-linolenic acid (ALA), eicosapentaenoic acid (EPA), docosahexaenoic acid (DHA), stearidonic acid (SDA), γ-linolenic acid (GLA), dihomo-γ-linolenic acid (DPA), and docosapentaenoic acid (DPA), particularly, DHA, ARA, and EPA, each of which may be present in free acid form, ionized or salt form, alkyl ester form, and/or triglyceride form. In some cases, the polyunsaturated fatty acids and/or long-chain fatty acids are present in triglyceride form.

Typically, the lipid formulation includes about 5% to about 35% by weight of an oil phase based on the total weight of the lipid emulsion. For example, the oil phase of the lipid emulsion is present in an amount of about 8% to 12%, of about 10% to about 20%, of about 10% to about 15%, of about 15% to about 20%, of about 12% to about 17%, of about 18% to 22% and/or about 20% by weight based on the total weight of the lipid formulation. The oil phase typically and preferably contains, in various amounts depending on the source of the oil, omega-3 fatty acids. The three types of omega-3 fatty acids involved in human metabolism are eicosapentaenoic acid (EPA) and docosahexaenoic acid (DHA), both of which are usually found in marine fish oils and α-linolenic acid (ALA), commonly found in plant oils.

The oil phase and its components can be derived from a single source or different sources (see, for example, Fell et al, Advances in Nutrition, 2015, 6(5), 600-610. Of the plant oils, currently used sources include, but are not limited to, soybean and olive oil as well as coconut or palm kernel oil (medium-chain triglycerides (MCTs)). Another source are algae, including microalgae such as Crypthecodinium cohnii and Schizochytrium sp., which in some cases serve as the single source of the long-chain polyunsaturated fatty acid docosahexaenoic acid (DHA). Marine oil used in parenteral lipid emulsions is processed from oily fish primarily found in cold water and including, but not limited to, herring, shad and sardines. However, other marine organisms can be used as an oil source, such as, for example, krill, such as Antarctic krill (Euphausia superba Dana). Krill oil, for example, provides for both EPA and DHA, in amounts of up to 35% w/w of the fatty acids. Krill oil as a component of lipid emulsions is considered to have anti-inflammatory properties due to the presence of DHA and EPA and is hypothesized to bind endotoxins (Bonaterra et al: Krill oil-in-water emulsions protects against lipopolysaccharides-induced proinflammatory activation of macrophages in vitro. Marine Drugs (2017), 15:74).

The lipid emulsions referred to herein may further include additional components, such as surfactants (also referred to as emulsifiers), co-surfactants, isotonic agents, pH adjusters, and antioxidants. Generally, surfactants are added to stabilize emulsions by reducing the interfacial tension between the oil phase and the aqueous phase. Surfactants typically include a hydrophobic part and a hydrophilic part, and the amount of surfactant/emulsifier included in the formulations is determined based on the amount that is needed to achieve a desired level of stabilization of the emulsion. Typically, the amount of surfactant in the lipid formulation is about 0.01 % to about 3% by weight based on the total weight of the lipid formulation, for example, about 0.01 % to about 2.5%, about 0.01 % to about 2.3%, about 0.02 % to about 2.2%, about 0.02 % to about 2.1%, about 0.02 % to about 2%, about 0.05% to about 1.8%, about 0.1% to about 1.6%, about 0.5% to about 1.5%, about 0.8% to about 1.4%, about 0.9% to about 1.3%, about 1% to about 1.2%, and/or about 1.2% by weight. Suitable surfactants and co-surfactants include surfactants that are approved for parenteral use, and include, but are not limited to, phospholipids (e.g., egg phosphatide and soy lecithin), oleate salts, and combinations thereof. Krill oil can also be used as an emulsifier in the lipid emulsion, wherein the lipid emulsion comprises about 0.5 to 2.2 wt % krill oil based on the total weight of the emulsion, and wherein the emulsion is free of egg yolk lecithin (US 2018/0000732 A1). Another exemplary surfactant is lecithin, including both natural and synthetic lecithin, such as lecithins derived from egg, corn or soybean or mixtures there-of. In some cases, lecithin is included in an amount of about 1.2% based on the total weight of the lipid formulation.

In embodiments, the lipid emulsions of the medical product of the invention, in particular the lipid emulsion comprising vitamin B12, should have a lipid component with a low peroxide level, preferably selected from the group consisting of soybean oil with low peroxides, olive oil, medium chain triglycerides, fish oil, fish oil extract, krill oil, algae oil, safflower oil, sunflower oil, corn oil, coconut oil, palm kernel oil, rapeseed oil, fungal oil and hydrogenated oils or mixtures thereof.

In embodiments, a lipid or oil with a low peroxide level has a peroxide value of no more than 5 milliequivalents (mEq) O₂/kg, preferably no more than 3 mEq O₂/kg, more preferably no more than 1.5 mEq O₂/kg. The oils listed above have been described as complying with this requirement. Furthermore, a person skilled in the are is able to identify low peroxide oils or lipids, such as oils with a peroxide value of no more than 5 milliequivalents (mEq) O₂/kg, by determining the peroxide value.

The peroxide value gives a measure of the extent to which an oil has undergone primary oxidation. The extent of secondary oxidation may be determined from p-anisidine test. The double bonds found in fats and oils play a role in autoxidation. Oils with a high degree of unsaturation are most susceptible to autoxidation. The best test for autoxidation (oxidative rancidity) is determination of the peroxide value. Peroxides are intermediates in the autoxidation reaction. Autoxidation is a free radical reaction involving oxygen that leads to deterioration of fats and oils. The peroxide value is defined as the amount of peroxide oxygen per 1 kilogram of fat or oil. Traditionally this was expressed in units of milliequivalents, and the SI unit is in millimoles per kilogram (N.B. 1 milliequivalents = 0.5 millimole; because 1 mEq of O₂ =1 mmol/2=0.5 mmol of O₂, where 2 is valence).

The peroxide value is determined by measuring the amount of iodine which is formed by the reaction of peroxides (formed in fat or oil) with iodide ion.

2I⁻ + H₂O + HOOH -> HOH + 2OH⁻ + I₂

The base produced in this reaction is taken up by the excess of acetic acid present. The iodine liberated is titrated with sodium thiosulphate.

2S₂O₃²⁻ + I₂ -> S₄O₆²⁻ + 2I⁻

The acidic conditions (excess acetic acid) prevents formation of hypoiodite (analogous to hypochlorite), which would interfere with the reaction. The indicator used in this reaction is a starch solution where amylose forms a blue to black solution with iodine and is colorless where iodine is titrated. A precaution that should be observed is to add the starch indicator solution only near the end point (the end point is near when fading of the yellowish iodine color occurs) because at high iodine concentration starch is decomposed to products whose indicator properties are not entirely reversible. Peroxide values of fresh oils are less than 10 milliequivalents O₂/kg; when the peroxide value is between 30* and 40 milliequivalents O₂/kg, a rancid taste is noticeable. As used herein, a low peroxide oil is an oil with no more than 5 milliequivalents O₂/kg, preferably no more than 3 mEq O2/kg, more preferably no more than 1.5 mEq O₂/kg.

Another method to determine the peroxide value, which can be used according to the invention, is based on the reduction of ferrous ions in acid solutions, wherein the amount of ferric product is measured as a xylenol orange complex at 560 nm. The method has been described, for example, in Gay et al: Hydroperoxide Assay with the Ferric-Xylenol Orange Complex. Analytical Biochemistry 273, 149-155 (1999). Generally, the hydroperoxides react with an excess of Fe²⁺ at low pH in the presence of the dye xylenol orange (XO) and the amount of Fe³⁺ generated is measured as the Fe-XO complex in the visible absorbance range.

Isotonic agents can be added to adjust the osmolarity of the lipid emulsion to a desired level, such as a physiologically acceptable level. Suitable isotonic agents include, but are not limited to, glycerol. Typically, the lipid emulsion formulation has an osmolarity of about 180 to about 300 milliosmols/liter, such as about 190 to about 280 milliosmols/liter, and/or about 200 to about 250 milliosmols/liter. In some cases, the lipid emulsion includes an isotonic agent in an amount of about 1% to about 10% by weight based on the total weight of the lipid formulation, such as about 1% to about 5%, about 1% to about 4%, and/or about 2% to about 3%. In some cases, the lipid emulsion formulation includes about 2% to about 3% by weight of glycerol.

pH modifiers can be added to adjust the pH to a desired level, such as a physiologically acceptable pH for parenteral use. Suitable pH modifiers include but are not limited to sodium hydroxide and hydrochloric acid. Typically, the lipid emulsions of the invention have a pH of about 5 to about 9, such as about 5.1 to about 8.9, about 5.2 to about 8.8, about 5.3 to about 8.0, about 5.4 to about 7.5, about 5.5 to about 7.0, about 5.5 to about 6.8, about 5.5 to about 6.3, about 5.6 to about 6.0, about 5.8, about 5.9 and/or about 6.0.

A lipid formulation and/or lipid emulsion of the medical product of the invention can include antioxidants. Suitable antioxidants may be pharmaceutically acceptable antioxidants and include, but are not limited to, EDTA, tocopherols (e.g., gamma tocopherol, delta tocopherol, alpha tocopherol), butylhydroxytoluol (BHT) or combinations thereof. In some cases, the lipid emulsions can include an antioxidant in an amount of about 0 to about 200 mg/L, for example, about 10 to about 200 mg/L, about 40 to about 150 mg/L, about 50 to about 120 mg/L, about 75 to about 100 mg/L antioxidant(s), such as vitamin E.

The aqueous (or water) used in preparing the product of the invention that are intended for intravenous application must conform to the pharmacopeial requirements that make it suitable for injection, that is, the water must be sterile water for injection.

The solutions and lipid emulsions of the medical product of the invention can be prepared according to generally known processes (see, for example, Hippalgaonkar et al, AAPS PharmSciTech 2010, 11(4), 1526-1540 or WO 2019/197198 A1)). Generally, water-soluble and oil-soluble ingredients are dissolved in the aqueous phase and oil phase, respectively.

If not defined otherwise herein, all terms used in the context of the present invention are to be interpreted according to the understanding of a person skilled in the art.

### FIGURES

The invention is further described by the following figures. These are not intended to limit the scope of the invention but represent preferred embodiments of aspects of the invention provided for greater illustration of the invention described herein.

### Description of the Figures:

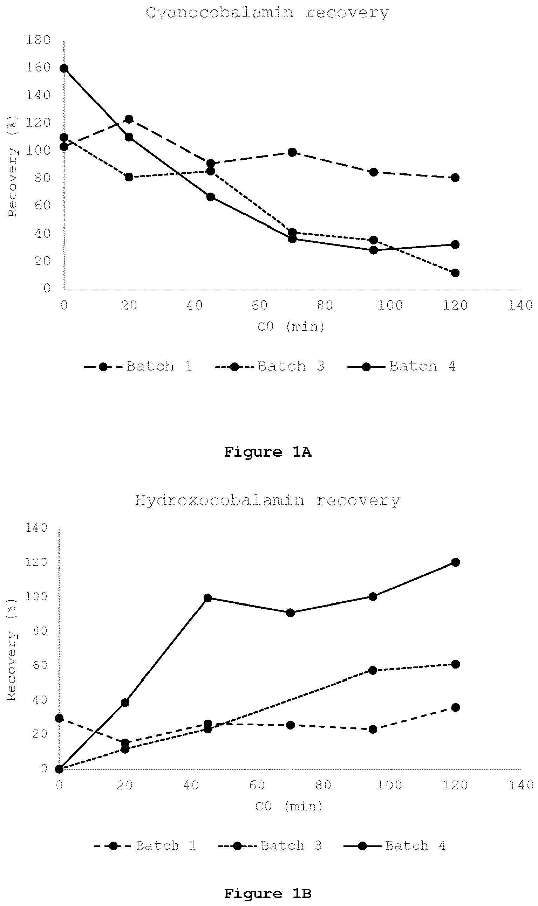

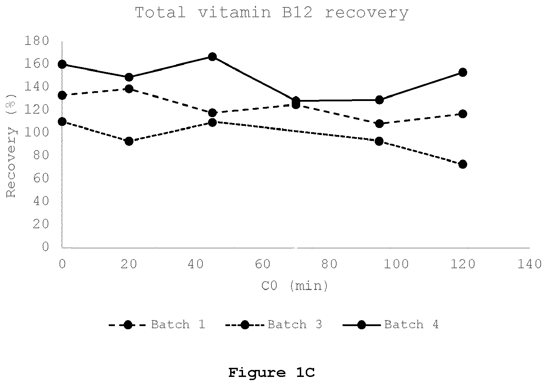

**Figure 1****:** Recovery of cyanocobalamin (Fig. 1A) and hydroxocobalamin (Fig. 1B) depending on Co. Also shown is the recovery of vitamin B12 in total, as a combination of hydroxocobalamin (Fig. 1C). Batches shown are further described in **Table 2.** With rising Co, recovery of cyanocobalamin decreases. At the same time, recovery of hydroxocobalamin increases as a result of the transformation of cyanocobalamin. The overall vitamin B12 recovery therefore remains relatively stable for a Co of about up to 120 minutes.

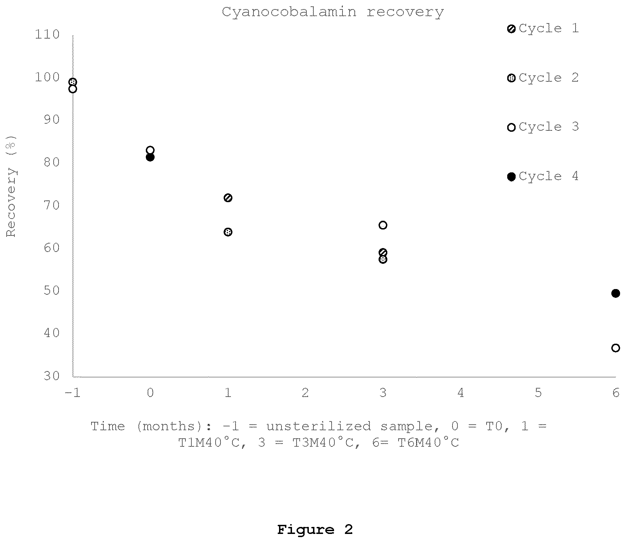

**Figure 2****:** Degradation of vitamin B12 when present in a standard parenteral nutrition glucose solutions and being submitted to sterilization with steam or water spray sterilization in volumes of 800 ml. Cycle 1 and Cycle 2 show sterilization with steam (see Table 6 and Tables 8A and B), whereas Cycles 3 and 4 show sterilization with water spray technology (see also Table 7 and Tables 9 A and B). Degradation of cyanocobalamin in the presence of glucose starts upon sterilization and rapidly progresses over the next months. Degradation is more pronounced when steam sterilization is used. In case of Cycles 1 and 2, after 6 months no cyanocobalamin can be detected anymore.

### EXAMPLES

The invention is further described by the following examples. These are not intended to limit the scope of the invention but represent preferred embodiments of aspects of the invention provided for greater illustration of the invention described herein.

In the following experiments, stability of vitamin B12 in various formulations, including formulations wherein vitamin B12 is comprised in lipid emulsions, were examined. Multiple studies have been performed, as summarized in following examples and tables.

The performed experiments in combination with review of the literature on the topic, lead to the result that vitamin B12, in particular in the form of cyanocobalamin is prone to interaction with certain trace elements in multi-vitamin mixtures, prone to interaction with ascorbic acid, prone to interaction with vitamin B1 (i.e. thiamine) degradation products (once heat sterilized), is both oxygen and light sensitive, is heat sensitive with a degradation proportional to Co (heat exposure), less stable in glucose based formulations both as cyanocobalamin and as hydroxocobalamin, and is also not stable in amino acid matrices as tested. It was otherwise found that vitamin B12 is turned into its first active degradation product, i.e. hydroxocobalamin, after standard heat sterilization processes especially in the presence of oxygen and/or other oxidizing agents. It was also found that vitamin B12 formulations are more stable at a pH around 5.9.

### Example 1: Stability of vitamin B12 during sterilization

Co and Fo are two quantitative parameters used to evaluate the amount of heat transferred to a product during a moist-heat sterilization process. Fo describes the lethality performance of the process, whereas Co, a parameter used to quantify the total heat consumption of a sample, relates to the chemical degradation of the API (Active Pharmaceutical Ingredient). Both parameters together provide for relevant information regarding sterilization processes and their impact on the ingredients of the solution to be sterilized. The ratio of Co vs Fo is therefore important to control heat degradation at defined lethality levels.

Therefore, in a first step, exemplary glucose solutions and lipid emulsions each containing vitamin B12 were submitted to different amounts of Co during the sterilization step to analyze the impact of Co on the stability of vitamin B12. Several Co values were targeted and a wide range, from 20 min to 120 min, was screened to understand the correlation between Co and the degradation of vitamin B12 in different media. Bags of lipid emulsion were produced, wherein the 5% olive-based emulsion contained vitamin B12 (cyanocobalamin form) at a concentration of 1dd in 25mL and, in one case, EDTA in a concentration of 0.1% w/v of the final emulsion (see **Table 2**). The glucose solutions used for the tests contained vitamin B12 in cyanocobalamin form or hydroxocobalamin form at a concentration of 1dd in 400mL. The heat sensitivity of the vitamin was assessed through the analysis of the respective degradation profile.

**Table 2: Summary of formulations tested for analyzing the stability of vitamin B12 under different sterilization conditions (C0)**

| **Batch** | **Batch 1** | **Batch 2** | **Batch 3** | **Batch 4** |
|---|---|---|---|---|
| **Solution** | Glucose A | Glucose B | Lipid Emulsion | Lipid Emulsion |
| **Concentration %** | 35% | 35% | 5% | 5% |
| **Vitamin** | B12 (cyanocobalamin) | B12 (hydroxocobalmin) | B12 (cyanocobalamin) | |
| **Excipient** | N/A | N/A | N/A | EDTA 0.1% w/v of final emulsion |
| **pH unsterilized** | 3.4 +/-0.1 | 3.4 +/-0.1 | 7.2 +/-0.2 | |
| **Bag material** | Multi-layer non-PVC film 100mL | Multi-layer non-PVC film 100mL | Multi-layer non-PVC film 100mL | Multi-layer non-PVC film 100mL |

Five (5) sterilization cycles at 123.5°C were used wherein five different Co values were targeted. For each cycle, bags of glucose with vitamin and bags with lipid emulsions comprising vitamin B12 were produced as described above in **Table 2** and used. Eight bags were used for each Batch at a C₀ of 20 min, 45 min, 70 min, 95 min and 120 min.

The solutions were analyzed unsterilized and sterilized for each cycle. Before each first cycle of the day, an empty cycle at 123.5°C for 15 minutes exposure was run. In that way, the heating phase of the cycle stayed equivalent for every cycle run. For the entire study, all bags used were multi-layer, non-PVC bags having a volume of 100 mL. The overpouching parameters were: vacuum = 85%; gas = 85%; sealing = 7.5 sec. All cycles were set up at 123.5°C.

Bags were then analyzed at the respective points in time to evaluate the impact on the degradation of vitamin B12. Results are provided in **Table 3.**

The quantitative determination of vitamin B12 in the form of cyano-and hydroxocobalamins was done by Ultra Performance Liquid Chromatography (UPLC), which separates components of a solution by partitioning of the analytes between a mobile liquid phase and a liquid stationary phase (reversed phase mode). The resulting difference in elution time allows to independently detect the different analytes. The system used was a Waters ACQUITY UPLC I-class coupled with a MS/MS Waters ESI Zspray detector. The column used was ACQUITY UPLC HSS T3 (1.8 µm, 2.1x100mm) from Waters. All solutions containing cyanocobalamin and hydroxocobalamin are extremely sensitive to light. Accordingly, all preparations containing these compounds were prepared freshly and kept in an amber volumetric flask. Also, the analysis was done diligently and without unnecessary interruptions. The mobile phases were mM ammonium bicarbonate (A1) and acetonitrile (25 mL of acetonitrile in 475 mL of water for chromatography) (B1). Standard stock solutions of cyanocobalamin and hydroxocobalamin were prepared in acetate buffer. Test solutions were prepared by diluting the samples with purified water (all in amber volumetric flasks). The gradient used was optimized to separate hydroxocobalamin and cyanocobalamin from other constituents of the matrix (Vit B2, B5, FSV, emulsion), see **Table 3.** The method is known in the art and has been described, for example, in Hampel et al, Journal of Chromatography B, 903, 7-12 (2012) or in Jiang et al., Waters LC/MS Analysis of Vitamin B12, Application NOTE, 2003.

**Table 3: Impact of Co (min.) on recovery rates for vitamin B12 for each batch before and after sterilization. Batches are designated according to the Batches shown in Table 2. "Target conc." refers to the concentration before sterilization. "US Recovery" refers to the recovery rate in the unsterilized probes. Vitamin B12 was used as cyanocobalamin ("CC") and as hydroxocobalamin ("HC"). "Sum B12" refers to the sum of CC and HC which could be recovered. "N/Q" means that no vitamin B12 could be recovered or was below the detection level.**

| **Batch** | **Vitamin** | **Target conc. (mg/L)** | **Unsterilized recovery (%)** | **20 min. recovery (%)** | **45 min. recovery (%)** | **70 min. recovery (%)** | **95 min. recovery (%)** | **120 min. recovery (%)** |
|---|---|---|---|---|---|---|---|---|
| 1 | **B12 CC** | **0.0125** | **103.2** | **123.2** | **91.2** | **99.2** | **84.8** | **80.8** |
| | B12 HC | N/A | 29.6 | 15.2 | 26.4 | 25.6 | 23.2 | 36.0 |
| | Sum B12 | 0.0125 | 132.8 | 138.4 | 117.6 | 124.8 | 108.0 | 116.8 |
| **2** | B12 CC | N/A | 3.9 | 10.9 | N/Q | N/Q | N/Q | N/Q |
| | **B12 HC** | **0.0128** | **115.6** | **116.4** | **33.6** | **20.3** | **18.8** | **N/Q** |
| | Sum B12 | 0.0128 | 115.6 | 116.4 | 33.6 | 20.3 | 18.8 | N/Q |
| 3 | **B12 CC** | **0.200** | **109.9** | **81.2** | **85.6** | **41.4** | **35.7** | **11.8** |
| | B12 HC | N/A | N/Q | 11.6 | 23.4 | no measurement | 57.2 | 61.0 |
| | Sum B12 | 0.200 | 109.9 | 92.8 | 109.0 | not applicable | 92.8 | 72.7 |
| 4 | **B12 CC** | **0.1998** | **159.8** | **110.0** | **66.8** | **36.8** | **28.4** | **32.5** |
| | B12 HC | N/A | N/Q | 38.8 | 99.6 | 91.2 | 100.4 | 120.4 |
| | **Sum B12** | **0.1998** | **159.8** | **148.8** | **166.4** | **128.0** | **128.8** | **153.0** |

As can be deduced from **Table 3,** vitamin B12 is clearly impacted by Co. **Figure 1** provides for a graphical representation of the values of **Table 3.** Generally, it was found that the hydroxocobalamin (HC) form increases when cyanocobalamin form decreases as a result of an increasing Co. It is assumed, therefore, that hydroxocobalamin is transformed into cyanocobalamin during the process. Hydroxocobalamin itself seems then to undergo degradation due to heat exposure.

In the lipid emulsion, Batch 3, this trend was also visible. Starting with about 110% recovery of cyanocobalamin for C₀=0min, it could only be recovered at a rate of about 12% for Co=120min. At the same time, hydroxocobalamin could not be detected when samples were not yet sterilized but had a recovery rate of 61% for C₀=120min. Results showed a degradation by about 35% of the total sum of vitamin B12 present at Co = 120min. It may further be assumed from the data that hydroxocobalamin is itself subject to degradation. Batch 4 follows the same trend of degradation for the two vitamin B12 forms. Cyanocobalamin decreased and hydroxocobalamin increased with an increasing Co. The total quantity of B12, however, remained relatively constant in Batch 4 which differed from Batch 3 with regard to the presence of EDTA as an excipient.

Glucose Batch 1 contained cyanocobalamin as vitamin B12 source. When Co increases, cyanocobalamin recovery decreases from 123% (Co = 20min) to 80% (Co = 120 min) while hydroxocobalamin recovery stays between 15.2% and 36%. The quantity of hydroxocobalamin was relatively constant. The total sum of vitamin B12 fluctuates between 108% and 138%. Vitamin B12 as cyanocobalamin accordingly seems to have a relatively good stability in glucose solutions. Indeed, it seems that Co has a somewhat lower impact on glucose solutions than on emulsions.

Glucose Batch 2 contained hydroxocobalamin (HC) as vitamin B12 source. Hydroxocobalamin recovery is 116% for Co = 20min, but strong degradation was observed from Co = 45min and HC has completely disappeared at Co = 120min. Accordingly, this test shows that hydroxocobalamin is heat sensitive and will get destroyed at certain sterilization conditions, and that working at carefully selected F0/C0 conditions is important for vitamin B12 stability in either form. It needs to be selected carefully for the intended terminally heat-sterilized multi-chamber container of the invention.

In a next step, the stability of vitamin B12 was examined in Glucose Batch 1 which seemed to be the most promising based on the results shown in **Table 3.** Accordingly, several bags were stored at 40°C/25%RH for 6 months (T6M). Batch Glucose 1 was analyzed at T6M to revisit the presence of vitamin B12 in either its cyanocobalamin or hydroxocobalamin form. In order to test the validity of the test, vitamins B1, B3 and B6 were tested together with vitamin B12 both after sterilization and then throughout shelf life. All of B1, B3 and B6 showed no significant degradation during the above sterilization. As shown in **Table 4,** vitamins B1, B3 and B6 were still stable at T6M with more than 80% recovery. However, vitamin B12 could not be detected at T6M despite having been present in Batch 1 after sterilization in sufficient amounts. Both Cyanocobalamin and hydroxocobalamin could not be detected, irrespective of the preceding Co value during sterilization. Accordingly, it was found that, surprisingly, even though glucose seems to be a viable medium for vitamin B12 during sterilization, it cannot be used for hosting vitamin B12 in either form. Accordingly, vitamin B12 cannot be stably provided in a glucose formulation and glucose should be avoided in a vitamin B12 formulation according to the invention.

**Table 4: Vitamin B12 (cyanocobalamin (CC) and hydroxocobalamin (HC)) recovery from glucose solution Batch A (see Tables 2 and 3) after storing the solution at a relative humidity (RH) of 25% and a temperature of 40°C for 6 months. A target concentration for HC is not provided because the starting material in each case was vitamin B12 in its cyanocobalamin form. "NQ" means that no vitamin B12 in its respective form could be detected.**

| **Batch** | **Vitamin** | **Target concentration (mg/L)** | **C0=45 min Recovery (%)** | **C0=70 min Recovery (%)** | **C0=120 min Recovery (%)** |
|---|---|---|---|---|---|
| **Glucose Batch 1** | **B1** | **19,1** | 94,2 | 93,2 | 92,4 |
| | **B3** | **100** | 94,6 | 93,5 | 90,7 |
| | **B6** | **18,2** | 84,9 | 82,4 | 80,8 |
| | **B12 CC** | **0,0125** | NQ | NQ | NQ |
| | **B12 HC** | NA | NQ | NQ | NQ |

### Example 2: Stability of vitamin B12 in glucose formulations, amino acid formulations and lipid emulsions during sterilization and storage

For testing the stability of vitamin B12 in formulation as they are typically used in parenteral nutrition, a 35% glucose solution, a 14.2% amino acid formulation such as found, for example, in Olimel N9E (Baxter Int. Inc.), and a 20% lipid emulsion such as found in Clinoleic 20% (Baxter Int. Inc.) were supplemented with different vitamins combinations which always included vitamin B12. KCl was added into the glucose solution to understand if it has an effect on the stability of vitamin B12. Vitamin B12 was introduced as thiamin HCl. Ascorbyl palmitate was added to the lipid emulsion formulation as a stabilizing agent due to its antioxidant properties. In addition, two separate micellar solutions ("Vit A" and "Vit B", see **Table 5)** comprising certain vitamins to be assessed with regard to degradation (B3, B6, B8, B9, B12, C) but formulated with different excipients as micellization agents were tested for vitamin B12 stability. Vit A consisted of a solution of mixed micelles with ascorbyl palmitate as a potential stability enhancer. Vit B was formulated with a mix of polysorbates, PEG and sorbitol to form micelles. The micellar solutions were prepared by mixing, under high speed, a solution composed of glycocholic acid and lecithins dissolved in water. In case of Vit B, the glycocholic acid and lecithin were replaced by PEG and polysorbate. All formulations were provided in standard flexible containers and overpouched with an aluminum overpouch for subsequent sterilization. For an overview over formulations and conditions see **Table 5.**

**Table 5: Summary of formulations tested for analyzing the stability of vitamin B12 under different conditions in glucose, amino acid and lipid emulsion formulations. Vitamins were added in an amount of "1 daily dose (dd)" each, e.g. 200 mg vitamin C, 5 µg vitamin B12 etc..**

| **Compartment** | **Glucose** 35% | **Amino Acid** 14.2% | **Emulsion** 20% | **Micelles Vit A** | **Micelles Vit B** |
|---|---|---|---|---|---|
| Micronutrients assessed for degradation | B3/B6/B12 | B8/B9/B12 | B12 | B8/B12/C | |
| Specific Excipients | KCl | With (Batch 5) and without EDTA/PVP17 (Batch 4) | Ascorbyl Palmitate | Ascorbyl palmitate | Polysorbate 80 & 20 PEG 400 Sorbitol |
| pH | 3.9-4.2 | 6.3-6.5 | 8.6-8.7 | 5.7-6.1 | NA |
| Vitamin concentration | 1dd/800 mL | 1dd/400 mL | 1dd/400 mL | 1dd/50 mL | 1dd/25 mL |

All samples were submitted to two different sterilization technologies. Steam sterilization targeting a Fo = 8 min was used for large volumes (400/800 ml, Cycle 2) and for small volumes (25/50 ml, Cycle 1) as lined out in **Table 5** above. The same samples were sterilized by water spray sterilization with a serial tower continuous sterilization equipment with two different exposure times (Cycles 3 (25 min.) and 4 (19 min.)).

The vitamin recovery was then compared between the different sterilization cycles and formulations to understand the impact of the formulation and the sterilization technology used on their respective short- and long-term stability. Therefore, after sterilization, the samples were stored at 40°C/25% RH for 6 months to evaluate the recovery of vitamin B12 at T1M, T3M and T6M. Besides, backup samples were stored at 5 °C. Finally, some samples were also stored at 25°C/40% RH to provide for real time stability data.

The results are shown in **Table 6A and B** and **Table 7A and B,** which reflect the outcome for the various sterilization approaches, respectively. The degradation of vitamin B12 in the glucose formulation when sterilized according to Cycles 1 through 4 is also shown in **Figure 2****.**

**Table 6A: Stability of vitamin B12 in glucose solutions sterilized according to Cycle 1 (steam sterilization), 25 ml. Vitamin B12 was not determined at T6M/40°C.**

| | **Target** | **Unsterilized** | | **Cycle 1** | | | | | | | |
|---|---|---|---|---|---|---|---|---|---|---|---|
| | | **T0** | | **T0** | | **T1M40°C** | | **T3M40°C** | | **T6M40°C** | |
| | **mg/L** | **mg/L** | **%** | **mg/L** | **%** | **mg/L** | **%** | **mg/L** | **%** | **mg/L** | **%** |
| **B3** | 49.954 | 51.10 | 102. 3 | N/A | N/A | 49.3 0 | 98. 7 | 50.2 0 | *100*. 5 | N/A | N/A |
| **B6** | 6.178 | 6.40 | 103. 7 | N/A | N/A | 6.40 | *103.* 7 | 6.50 | *105.* 3 | N/A | N/A |
| **B12** | 0.0063 | 0.006 2 | 99.0 | N/A | N/A | **0.00 45** | ***71.9*** | **0.00 37** | ***59.1*** | N/A | N/A |

**Table 6B: Stability of vitamin B12 in glucose solutions sterilized according to Cycle 2 (steam sterilization), 800 ml. Vitamin B12 was not determined at T6M/40°C.**

| | **Target** | **Unsterilized** | | **Cycle 2** | | | | | | | |
|---|---|---|---|---|---|---|---|---|---|---|---|
| | | **T0** | | **T0** | | **T1M40°C** | | **T3M40°C** | | **T6M40°C** | |
| | **mg/L** | **mg/L** | **%** | **mg/L** | **%** | **mg/L** | **%** | **mg/L** | **%** | **mg/L** | **%** |
| **B3** | 49.954 | 51.10 | 102.3 | N/A | N/A | 50.6 0 | 101.3 | 51.2 0 | 102. 5 | N/A | N/A |
| **B6** | 6.178 | 6.40 | 103.7 | N/A | N/A | 5.93 | 96.1 | 6.00 | 97.2 | N/A | N/A |
| **B12** | 0.0063 | 0.0062 | 99.0 | N/A | N/A | **0.00 40** | **63.9** | **0.00 36** | **57.5** | N/A | N/A |

**Table 7A: Stability of vitamin B12 in glucose solutions sterilized according to Cycle 3 (water spray sterilization, Co=25 minutes), 800 ml. The asterisk indicates that the value is the sum of cyanocobalamin and hydroxocobalamin.**

| | **Target** | **Unsterilized** | | **Cycle 3** | | | | | | | |
|---|---|---|---|---|---|---|---|---|---|---|---|
| | | **TO** | | **TO** | | **T1M40°C** | | **T3M40°C** | | **T6M40°C** | |
| | mg/L | mg/L | % | mg/L | % | mg/L | % | mg/L | % | mg/L | % |
| **B3** | 49.954 | 52.80 | 105.7 | 50.50 | *101*. *1* | N/A | N/A | 50.30 | *100*. *7* | 48.27 | *96*. *6* |
| **B6** | 6.178 | 6.61 | 107.1 | 6.23 | *101*. *0* | N/A | N/A | 6.10 | *98.9* | 6.04 | *97.9* |
| **B12** | 0.0063 | 0.006 | 97.4 | **0.005 1** | ***81*.*4*** | **N/A** | **N/A** | **0.00 41** | ***65.5*** | **0.002 3*** | ***36. 7**** |

**Table 7B: Stability of vitamin B12 in glucose solutions sterilized according to Cycle 4 (water spray sterilization, C₀=19 minutes), 800 ml. The asterisk indicates that the value is the sum of cyanocobalamin and hydroxocobalamin.**

| | **Target** | **Unsterilized** | | **Cycle 4** | | | | | | | |
|---|---|---|---|---|---|---|---|---|---|---|---|
| | | **TO** | | **TO** | | **T1M40°C** | | **T3M40°C** | | **T6M40°C** | |
| | mg/L | mg/L | % | mg/ L | % | mg/ L | % | mg/ L | % | mg/ L | % |
| **B3** | 49.954 | 52.80 | 105.7 | 50.40 | *100*.*9* | N/A | N/A | 51.00 | *102.1* | 49.35 | *98.8* |
| **B6** | 6.178 | 6.61 | 107.1 | 6.27 | *101*.*6* | N/A | N/A | 5.90 | *95.6* | 6.03 | *97*.*7* |
| **B12** | 0.0063 | 0.006 | 97.4 | **0.0052** | ***83.0*** | **N/A** | **N/A** | **0.0041** | ***65.5*** | **0.0031*** | ***49.5**** |

It was found that vitamins B3 and B6, for example, are stable in the glucose solutions tested. No significant degradation was detected after steam sterilization and throughout storage. Vitamin B12, in contrast, showed a significant degradation over time, irrespective of the volumes and sterilization techniques used, and despite having a favorable Co value (Cycle 3) where water spray sterilization was used. In summary it was found that despite potentially promising stability rates in glucose upon sterilization, glucose formulations such as used for PN cannot be used to stably provide vitamin B12 in terminally heat sterilized medical products for parenteral nutrition.

In addition **(Table 5),** vitamin B12 stability was tested in amino acid formulations as they are used in parenteral nutrition. Vitamin B12 proved to be similarly prone to degradation in the amino acid formulations (not shown). EDTA/PVP17 resulted in a less pronounced degradation, but vitamin B12 (cyanocobalamin) recovery rate was still only 78.9% at T6M40°C after steam sterilization. Without EDTA/PVP17, the recovery rate for vitamin B12 (cyanocobalamin) was only 60.2% at T6M40°C after steam sterilization.

Vitamin B12 was also considerably degraded in the lipid emulsion **(Tables 8** through **11).** Lipid emulsions are generally part of 3-chamber-bags for parenteral nutrition and would therefore be another option to provide vitamin B12, as the amino acid and glucose formulations proved to be not viable for stably providing the vitamin. As shown in **Table 5,** an emulsion as it is used today in parenteral nutrition was provided and steam sterilized or sterilized by water spray sterilization with a serial tower continuous sterilization equipment as described before (see Cycles 1 and 2 and 3 and 4, respectively). The instability of vitamin B12 in the standard lipid emulsions is probably linked to the presence of ascorbyl palmitate, which is generally present as an antioxidant. It should thus be avoided in formulations according to the invention.

**Table 8: Recovery rates for vitamin B12 when provided in a lipid emulsion according to Table 5 and sterilized by steam sterilization, Cycle 1. "LOD" means "level of detection".**

| | **Target** | **Unsterilized** | | **Cycle 1** | | | | | | | |
|---|---|---|---|---|---|---|---|---|---|---|---|
| | | **T0** | | **T0** | | **T1M40°C** | | **T3M40°C** | | **T6M40°C** | |
| | mg/L | mg/L | % | mg/L | % | mg/L | % | mg/L | % | mg/L | % |
| **B12** | 0.0125 | 0.015 | 118. 0 | NA | NA | **0.004** | **35.3** | **<LOD** | NA | NA | NA |

**Table 9: Recovery rates for vitamin B12 when provided in a lipid emulsion according to Table 5 and sterilized by steam sterilization, Cycle 2.**

| | **Target** | **Unsterilized** | | **Cycle 2** | | | | | | | |
|---|---|---|---|---|---|---|---|---|---|---|---|
| | | **T0** | | **T0** | | **T1M40°C** | | **T3M40°C** | | **T6M40°C** | |
| | mg/L | mg/L | % | mg/L | % | mg/L | % | mg/L | % | mg/L | % |
| **B12** | 0.0125 | NA | NA | **0.00 5** | **36 .9** | **<LOD** | NA | NA | NA | NA | NA |

**Table 10: Recovery rates for vitamin B12 when provided in a lipid emulsion according to Table 5 and sterilized by water spray sterilization. The asterisk indicates that the value is the sum of cyanocobalamin and hydroxocobalamin.**

| | **Target** | **Unsterilized** | | **Cycle 3** | | | | | | | |
|---|---|---|---|---|---|---|---|---|---|---|---|
| | | **T0** | | **T0** | | **T1M40°C** | | **T3M40°C** | | **T6M40°C** | |
| | mg/L | mg/L | % | mg/L | % | mg /L | % | mg/L | % | mg/L | % |
| **B12** | 0.0125 | 0.014 | *110*. *0* | **0.005** | ***43.4*** | **NA** | **NA** | **NA** | ***NA*** | **0.00 37*** | *29.5* ***** |

**Table 11: Recovery rates for vitamin B12 when provided in a lipid emulsion according to Table 5 and sterilized by water spray sterilization with a serial tower continuous sterilization equipment. The asterisk indicates that the value is the sum of cyanocobalamin and hydroxocobalamin.**

| | **Target** | **Unsterilized** | | **Cycle 3** | | | | | | | |
|---|---|---|---|---|---|---|---|---|---|---|---|
| | | **TO** | | **TO** | | **T1M40°C** | | **T3M40°C** | | **T6M40°C** | |
| | mg/L | mg/L | % | mg/L | % | mg /L | % | mg/L | % | mg/L | % |
| **B12** | 0.0125 | 0.014 | *110*. *0* | **0.006** | ***51.4*** | **NA** | **NA** | **NA** | ***NA*** | **0.00 55*** | ***44.2* *** |

In consequence, vitamin B12 proved to be instable over time also in lipid emulsions in presence of ascorbyl palmitate as they are generally provided in PN. Finally, as a further option, micellar solutions were tested as potential formulation for stably providing vitamin B12 in medical products for parenteral nutrition.

Accordingly, mixed micelles solutions having smaller volumes (25ml or 50 ml, see **Table 5)** were produced according to known methods. The micellar solutions comprised the same vitamins (i.e. B8, B12, C) but were formulated with different excipients as micellization agents. Vit A consisted of a solution of mixed micelles with ascorbyl palmitate as a potential stability enhancer. Vit B was formulated with a mix of agents **(Table 5)** to form micelles as known in the art. The mixed micelles solutions from Table 5 were then sterilized according to Cycles 1, 2, 3 and 4 as described before and the degradation of vitamin B12 during sterilization and subsequent storage was monitored as described before.

It was found that sterilization with either steam or water spray and subsequent storage lead to an almost complete degradation of vitamin B12 in both mixed micelle solutions tested. In summary, also mixed micelle solutions as known from the art, proved to be not optimal for stably providing vitamin B12 in a terminally heat sterilized medical product for parenteral nutrition.

### Example 3: Preparation of formulations comprising vitamin B12

As all generally available formulations provided in PN products, such as glucose, amino acid, or lipid emulsion formulations, including mixed micelle solutions, proved to be detrimental or at least unfavorable for the stable addition of vitamin B12 throughout sterilization and storage, specific vitamin B12 formulations were developed and the stability of vitamin B12 was tested in said dedicated formulations. **Table 12** provides for the various batches which were prepared for testing the stability of vitamin B12 in each setting.

For Batches 1 to 3, concentrated emulsions (40%) were diluted with a factor of 8. The vitamin bulk was used to dilute the emulsion from 40% to 5%. The pH was adjusted at 5.9 with a citrate buffer for Batch 1 and a phosphate buffer for Batch 2. An adjustment to pH 6.5 was performed for Batch 3 with HCl. The final volume was provided by adding Milli-Q water. In case of Batches 2 and 3, EDTA and PVP K17 were added before filling up to the final volume. The emulsions were filtered with a 4.5µm filter before filling. Flushing of the solutions using N₂ was performed before starting the filling of each batch. The Batches were manufactured under exclusion of light and oxygen. The dissolved oxygen was measured at the beginning of the filling to ensure it remains <1.0 ppm. The headspace volume was set as small as possible (≤10 mL) and the filling volume limits was 100mL per monobag, ± 2 mL. To avoid any risk of vitamin degradation, an aluminum overpouch was used to protect each monobag. An oxygen absorber was placed between primary bag and overpouch. Seals were inspected before sterilization rack loading. Four samples per Batch were kept unsterilized for Batches 1 through 4. The other bags were sterilized by steam sterilization in an autoclave to achieve a C0 of 77 min at the end of the exposure phase. **Table 12** provides for the composition of the four batches which were tested.

**Table 12: Outline of test batches comprising various formulations with vitamin B12. The batches differed with regard to the presence of oil, stabilizing agents and pH adjustment agents, respectively. Vitamin B12 was added as cyanocobalamin.**

| **Batch ID** | **Description** | **pH adjustment agent** |
|---|---|---|
| 1 | Emulsion (5% olive oil) + B12 | Citrate buffer 5mM |
| 2 | Emulsion (5% olive oil) + B12 + EDTA/PVP | Phosphate buffer 5mM |

| **Batch ID** | **Description** | **pH adjustement agent** |
|---|---|---|
| 3 | Emulsion (5% olive oil) + B12 + EDTA/PVP | NaOH/HCl |
| 4 | Aqueous + B12 + EDTA/PVP | NaOH/HCl |

**Table 13: Description of Batches 1 to 4 which were tested for vitamin B12 stability.**

| | | **Concentration (mg/L) corresponding to 1dd/25mL** | | | |
|---|---|---|---|---|---|
| **Constituents** | **Daily dose (dd)** | **Batch 1** | **Batch 2** | **Batch 3** | **Batch 4** |
| **Vitamins tested** | | | | | |
| Vitamin B12 (cyanocobalamin) | 5 µg | 0.2 | 0.2 | 0.2 | 0.2 |

| **Excipients** | | | | | |
|---|---|---|---|---|---|
| Olive oil | N/A | X | X | X | - |
| Egg lecithin | N/A | X | X | X | - |
| Glycerol | N/A | X | X | X | - |
| Sodium Oleate | N/A | X | X | X | - |
| Monosodium Phosphate | N/A | - | 5 mM | - | - |
| Acid citric | N/A | 5 mM | - | - | - |
| EDTA | N/A | - | 0.01% w/v | 0.01% w/v | 0.01% w/v |
| PVP 17 | N/A | - | 0.05% w/v | 0.05% w/v | 0.05% w/v |
| Sodium hydroxide, hydrochloric acid | N/A | pH 5.9 | | pH 6.5 | pH 5.9 |

The result for the emulsion Batches 1 to 3 are provided below in **Table 14.** It provides for the results of vitamin B12 recovery for cyanocobalamin (CC), hydroxocobalamin (HC) before and directly after water cascade sterilization. The sum of both vitamin forms is provided as well. Also tested were the pH after sterilization and the dissolved oxygen content (DO) in ppb.

**Table 14: Vitamin B12 recovery, pH and dissolved oxygen content for Batches 1 to 3 (5% emulsions) before and after sterilization. Cyanocobalamin (CC) and hydroxocobalamin (HC) were determined and the sum of both vitamin B12 forms is shown.**

| **Vitamins / Others** | | **B12 (CC)** | **B12 (HC)** | **B12 Sum** | **pH** | **DO (ppb)** |
|---|---|---|---|---|---|---|
| **Batch** | **Emulsions** | **106.9** | **112.9** | N/A | N/A | N/A |
| 1 | Unsterilized | 113.8 | 15.2 | 129.0 | N/A | N/A |
| 1 | Sterilized | 63.2 | 12.6 | 75.8 | 6.22 | 0.0 |
| 2 | Unsterilized | 110.8 | 14.5 | 125.3 | N/A | N/A |
| 2 | Sterilized | **75.0** | **49.4** | **124.4** | **5.99** | **0.0** |
| 3 | Unsterilized | 115.1 | 12.5 | 127.6 | N/A | N/A |
| 3 | Sterilized | 64.3 | 13.5 | 77.8 | 6.50 | 0.0 |

Recovery of vitamin B12 was lower for Batch 1 compared to Batch 2 (containing phosphate buffer, EDTA and PVP) and Batch 3 (containing EDTA and PVP), indicating that the phosphate buffer and the presence of EDTA and PVP K17 attributed to the higher stability of vitamin 12 in Batches 2 and 3. Importantly and surprisingly, the sum of the vitamin B12 forms indicates that hydroxocobalamin is not degraded further in the 5% emulsions. Accordingly, the total amount of vitamin B12 remains stable and the tested emulsions seem to be a viable form of stably providing vitamin B12 in a terminally heat-sterilized multi-chamber container according to the invention.

The formulation of vitamin B12 (Batch 4) was tested in optimized conditions (oxygen content, pH, sterilization method), and the results are summarized in **Table 15.** Vitamin B12 remains stable up to T7M40°C (seven months at 40°C). Vitamin B12 was stable and present as cyanocobalamin.

**Table 15: Results for Batch 4 (aqueous solution): vitamin B12 recovery, pH and dissolved oxygen content before and after sterilization as well as after storage for 3 and 7 months at the indicated temperatures. ND means "not detected".**

| **Batches** | | **Conditions** | **Sum B12** | **B12 (CC)** | **B12 (HC)** | **pH** | **DO (ppb)** |
|---|---|---|---|---|---|---|---|
| **Analysis 1** | | | | **108.4** | **134.1** | | |
| 4 | Unsterilized | T0 | 107.0 | 107.0 | ND | - | 0 |
| 4 | Sterilized | T0 | 107.4 | 97.8 | 9.6 | 6.01 | 0 |

| **Analysis 2** | | | | **112.9** | **114.1** | | |
|---|---|---|---|---|---|---|---|
| 4 | Sterilized | T3M 40°C | 94.8 | 94.8 | ND | 5.9 | 0.025 |
| 4 | Sterilized | T7M 40°C | 116.1 | 116.1 | ND | - | - |
| 4 | Sterilized | T7M 25°C | 125.7 | 125.7 | ND | - | - |

### Example 3: Impact of oxygen and light on the stability of vitamin B12

A 10% soybean oil emulsion with low peroxide level (1 mEq O₂/kg) was formulated to contain vitamin B12 in its cyanocobalamin form (1dd/25mL). The pH was adjusted to an optimum of 5.9 with a phosphate buffer. In addition, an aqueous solution was prepared and (1dd/25mL) vitamin B12 was added. The pH was also adjusted to 5.9 using HCl. Various oxygen exposure levels were tested.
(a) 0 ppm oxygen: packaging of the formulation into an oxygen permeable flexible container, which was overpouched in the presence of an oxygen absorber. Like this, the oxygen contained in the formulation is removed.
(b) <0.5 ppm oxygen: packaging into an oxygen barrier material after flushing of the bag with N₂, followed by overpouching without oxygen absorber. The oxygen in the formulation remains entrapped therein.
(c) <1.0 ppm oxygen: packaging into oxygen barrier material followed by overpouching without oxygen absorber. Oxygen remains entrapped in the formulation.

The various formulations were exposed to light for 0, 2h, 7h, 24h.

For the lipid emulsion it was found that at the chosen conditions (10% low peroxide lipid emulsion, pH 5.9, phosphate buffer, oxygen remains below 1.0 ppm) it was possible to stabilize vitamin B12 in a way that surprisingly it is less impacted by light and oxygen as it could be expected based on what is known about light sensitivity of the vitamin. In contrast, cyanocobalamin over time is transformed into hydroxocobalamin, but the sum of both forms falls below a recovery of 80% only after 5 months at 40°C.

In contrast, vitamin B12 in the aqueous solution remains light sensitive (see **Figure 3**). When exposed to light for more than 2 hours the recovery of vitamin B12 drops below 80% very quickly.

### Example 4: Impact of pH on the stability of vitamin B12

Two batches of a lipid emulsion (5% olive oil comprising 1 dd/25 mL vitamin B12 (associated vitamins A,D,E,K) were filled in oxygen permeable bag material with an oxygen absorber, protected from light and stored for 6 months at 40°C after sterilization (Co=40min for Batch 1 and C0=80min for Batch 2). Batch 1 was adjusted at pH 7.0 whereas Batch 2 was adjusted to pH 5.9. 53.8% of vitamin B12 (cyanocobalamin plus hydroxocobalamin) were recovered from Batch 1, whereas 138% vitamin B12 (cyanocobalamin plus hydroxocobalamin) were recovered from Batch 2.

## Claims

1. A terminally heat sterilized multi-chamber container for parenteral nutrition, comprising a first chamber containing a macronutrient formulation and a second chamber comprising a vitamin B12 formulation, wherein the vitamin B12 formulation
(a) is essentially free from glucose, amino acids, vitamin B1, ascorbyl palmitate and/or vitamin C;
(b) stably comprises vitamin B12 for at least 6 months at a temperature of from 1°C to 40°C and/or for at least 24 months at a temperature of 25°C.

2. The terminally heat-sterilized multi-chamber container according to claim 1, wherein the reconstituted volume is more than 100 mL.

3. The terminally heat-sterilized multi-chamber container according to claim 1 or claim 2, wherein the vitamin B12 formulation contains no more than 1.5 ppm dissolved oxygen (DO).

4. The terminally heat-sterilized multi-chamber container according to any of claims 1 to 3, wherein the vitamin B12 formulation is protected from light during preparation, filling, sterilization and storage.

5. The terminally heat-sterilized multi-chamber container according to any of claims 1 to 4, wherein the vitamin B12 formulation is an aqueous solution.

6. The terminally heat-sterilized multi-chamber container according to any of claims 1 to 4, wherein the vitamin B12 formulation is a lipid emulsion comprising an aqueous phase and from 1% to 20% by weight of an oil phase based on the total weight of the lipid emulsion.

7. The terminally heat-sterilized multi-chamber container according to any of claims 1 to 6, wherein vitamin B12 is provided in the form of cyanocobalamin and/or hydroxocobalamin.

8. The terminally heat-sterilized multi-chamber container according to any of claims 1 to 7, wherein the pH of the vitamin B12 formulation is in the range of 5-8, preferably 5-7.

9. The terminally heat-sterilized multi-chamber container according to any of claims 1 to 8, wherein the vitamin B12 formulation does not comprise trace elements.

10. The terminally heat-sterilized multi-chamber container according to any of claims 1 to 9, wherein the terminal heat sterilization is done by superheated water sterilization.

11. The terminally heat-sterilized multi-chamber container according to any of claims 1 to 10, wherein the terminal heat sterilization is performed at a temperature of from 100°C to 122°C for 10 min to 60 min.

12. The terminally heat-sterilized multi-chamber container according to any of claims 1 to 11, wherein the container consists of a flexible polymeric material which comprises
(a) an oxygen barrier film, preferably with an oxygen permeability of less than 50 cc/m²/day, and/or
(b) an inner lining containing a polyolefin, wherein the inner lining is free of polyvinyl chloride (PVC), plasticizers, adhesives or latex.

13. The terminally heat-sterilized multi-chamber container according to any of claims 1 to 12, wherein the vitamin B12 formulation further comprises at least one B vitamin selected from the group consisting of vitamin B2 (riboflavin), vitamin B3 (niacin), vitamin B5 (pantothenic acid), vitamin B6 (pyroxidine), vitamin B7 (biotin) and vitamin B9 (folate) or mixtures thereof.

14. The terminally heat-sterilized multi-chamber container according to any of claims 1 to 13, wherein the vitamin B12 formulation is a lipid emulsion according to claim 5 and further comprises at least one vitamin selected from the group consisting of vitamin A, retinyl palmitate, retinyl stearate, vitamin E, gamma-tocopherol, alpha-carotene, trans-beta-carotene, cis-beta-carotene, beta-cryptoxanthin, lutein, zeaxanthin, trans-lycopene, cis-lycopene, vitamin D, vitamin K and mixtures thereof.

15. The terminally heat-sterilized multi-chamber container according to any of claims 6 to 14, wherein the oil phase comprises or consists of a low peroxide level oil as a lipid component with a peroxide value of no more than 5 milliequivalents (mEq) O₂/kg, preferably no more than 3 mEq O₂/kg, more preferably no more than 1.5 mEq O2/kg, such as soybean oil with low peroxides (refined soybean oil), olive oil, medium chain triglycerides, fish oil, fish oil extract, krill oil, algae oil, safflower oil, sunflower oil, corn oil, coconut oil, palm kernel oil, rapeseed oil, fungal oil and hydrogenated oils or mixtures thereof.

16. The terminally heat-sterilized multi-chamber container according to any of claims 6, 14 or 15, wherein the oil phase does not comprise ascorbyl palmitate.

17. The terminally heat-sterilized multi-chamber container according to any of claims 1 to 16, wherein the vitamin B12 formulation comprises one or more stabilizing agents selected from the group comprising or consisting of
(a) EDTA, preferably at a concentration of no more than 0.1 g/L;
(b) monobasic sodium phosphate buffer, preferably at a concentration of no more than 0.5 mM.

18. The terminally heat-sterilized multi-chamber container according to any of claims 1 to 17, wherein the vitamin B12 formulation has a volume of from 2 to 100 ml, preferably from 5 to 50 ml, more preferably from 10 to 30 ml and most preferably from 15-25 ml.

19. The terminally heat-sterilized multi-chamber container according to any of claims 1 to 18, wherein the vitamin B12 formulation comprises 0.5 µg to 10 µg of vitamin B12.

20. The terminally heat-sterilized multi-chamber container according to any of claims 1 to 19, wherein the container has at least two, at least three, at least four, at least five or at least six chambers.

21. The terminally heat-sterilized multi-chamber container according to any of claims 1 to 20, wherein the container comprises a first chamber comprising a carbohydrate formulation, a second chamber comprising an amino acid formulation, a third chamber comprising a lipid emulsion formulation and a fourth chamber comprising the vitamin B12 formulation.

22. A method of preparing a terminally heat-sterilized multi-chamber container according to any of claims 1 to 5, claims 7 to 13, and claims 17 to 21, wherein the vitamin B12 formulation is an aqueous solution, the method comprising the steps of
(a) providing an aqueous medium suitable for injection, preferably water for injection;
(b) adjusting the aqueous solution to a concentration of dissolved oxygen (DO) of no more than 1 ppm and a pH value of 5-8, preferably 5 to 7,
(c) dissolving vitamin B12, preferably in the form of cyanocobalamin, in the low DO solution of step b), and
(d) sterilizing the solution by heat sterilization using water spray or water cascade sterilization.

23. The method according to any of claims 1 to 4 and 6 to 21, wherein the vitamin B12 formulation is a lipid emulsion, the method comprising the steps of
(a) providing an aqueous solution suitable for injection, preferably water for injection,
(b) optionally providing a lipid phase including adding at least one fat-soluble vitamin and/or nutrient selected from the group consisting of vitamin A, retinyl palmitate, retinyl stearate, vitamin E, gamma-tocopherol, alpha-carotene, trans-beta-carotene, cis-beta-carotene, beta-cryptoxanthin, lutein, zeaxanthin, trans-lycopene, cis-lycopene, vitamin D, vitamin K and mixtures thereof to the lipid phase,
(c) separately heating up the lipid phase and the aqueous solution, preferably to a temperature of from 60°C to 90°C, under agitation;
(d) preparing the pre-emulsion by transferring the oil phase to the aqueous solution under agitation;
(e) homogenizing the pre-emulsion at a temperature of from 40°C to 60°C under pressure;
(f) optionally adding water to adjust the required volume and concentrations;
(g) adjusting the lipid emulsion to a concentration of dissolved oxygen (DO) of no more than 1 ppm and a pH value of 5-9;
(h) dissolving vitamin B12, preferably in the form of cyanocobalmin, in the low DO lipid emulsion, optionally together with additional B-vitamins; and
(i) sterilizing the solution, preferably by heat sterilization, more preferably by heat sterilization using water spray or water cascade sterilization.
